# EUROPÄISCHE PATENTSCHRIFT

(11) **EP 1 833 536 B1**
(45) Veröffentlichungstag und Bekanntmachung des Hinweises auf die Patenterteilung: **13.05.2020**
(21) Anmeldenummer: 05804308.4
(22) Anmeldetag: 30.11.2005
(51) Int. Cl.: A61M 5/20, A61M 5/315, A61M 5/24, A61M 5/32, A61M 5/31, F04B 49/10

(54) **VORRICHTUNG ZUR DOSIERTEN VERABREICHUNG EINES FLUIDEN PRODUKTS MIT KUPPLUNG**
DEVICE FOR THE DOSED ADMINISTRATION OF A FLUID PRODUCT, PROVIDED WITH A COUPLING
DISPOSITIF D'ADMINISTRATION D'UNE DOSE DE PRODUIT FLUIDE POURVU D'UN ACCOUPLEMENT

(30) Priorität: 31.12.2004 DE 102004063647
(43) Veröffentlichungstag der Anmeldung: 19.09.2007
(73) Patentinhaber: TecPharma Licensing AG, 3401 Burgdorf (CH)
(72) Erfinder: FIECHTER, Patrick, CH-4950 Huttwil (CH); KOHLBRENNER, Philippe, CH-3413 Kaltacker (CH); MEIER, Christoph, CH-3427 Utzenstorf (CH); WIDMER, Urs, CH-3013 Bern (CH); WITTMANN, Juergen, CH-3400 Burgdorf (CH); KUENZLI, Daniel, CH-4513 Langendorf (CH); STETTLER, Peter, CH-3422 Kirchberg (CH)
(86) Internationale Anmeldenummer: PCT/CH2005/000713
(87) Internationale Veröffentlichungsnummer: WO 2006/069457

(56) Entgegenhaltungen:
- EP-A- 0 295 075
- EP-A- 0 498 737
- WO-A-02/092153
- WO-A-2004/078239
- WO-A-2004/078240
- US-B1- 6 364 860

## Beschreibung

Die Erfindung betrifft eine Vorrichtung zur dosierten Verabreichung eines fluiden Produkts. Die Vorrichtung kann insbesondere ein Injektionsgerät sein, vorzugsweise in der Form eines Injektionspens. Injektionsgeräte sind beispielsweise aus der Diabetestherapie, der Verabreichung von Wachstumshormonen oder Osteoporosepräparaten bekannt. Derartige Geräte sollen einerseits die Gewähr dafür bieten, dass die richtige Dosis verabreicht wird, andererseits sollen die Geräte einfach und bequem bedienbar sein, zwei Forderungen, die insbesondere von Bedeutung sind, wenn der Benutzer sich das betreffende Produkt selbst verabreicht.

Ein aus der WO 01/10484 A1 bekanntes Injektionsgerät weist eine Förder- und Dosiereinrichtung auf mit einer Kolbenstange, einem Antriebsglied und einer zweigliedrigen Kupplung bestehend aus einem Kupplungseingangsglied und einem Kupplungsausgangsglied. Das Antriebsglied ist mit einem Gehäuse des Injektionsgeräts in einem Gewindeeingriff und über das Kupplungseingangsglied mit dem Kupplungsausgangsglied gekoppelt. Das Kupplungsausgangsglied ist mit der Kolbenstange in einem weiteren Gewindeeingriff. Die Kolbenstange wird von dem Gehäuse verdrehgesichert axial linear geführt. Für die Einstellung einer Dosis verdreht der Benutzer das Antriebsglied, das wegen des Gewindeeingriffs mit dem Gehäuse daraufhin in die proximale Richtung aus dem Gehäuse ausfährt. Bei dieser kombinierten Translations- und Rotationsbewegung nimmt das Antriebsglied das Kupplungseingangsglied mit. Das Kupplungseingangsglied ist auf dem Kupplungsausgangsglied verdrehgesichert linear geführt und bildet mit dem Antriebsglied eine Ratsche, die eine Drehbewegung des Antriebsglieds relativ zu dem Kupplungseingangsglied nur in eine Richtung zulässt. Nach Einstellung der gewünschten Dosis wird diese ausgeschüttet, indem der Benutzer das Antriebsglied in die distale Richtung in das Gehäuse drückt. Aufgrund des Gewindeeingriffs mit dem Gehäuse ist der Translationsbewegung des Antriebsglieds eine Rotationsbewegung überlagert, die über das Kupplungseingangsglied auf das Kupplungsausgangsglied übertragen wird. Die Rotationsbewegung des Kupplungsausgangsglieds bewirkt den Vortrieb der Kolbenstange und damit die Produktausschüttung. Umständlich gestaltet sich allerdings die Korrektur einer zu hoch eingestellten Dosis. Wurde bei der Einstellung nämlich die gewünschte Dosis überschritten, muss das Antriebsglied bis in eine der Maximaldosis entsprechenden Position verdreht werden. Das bei der kombinierten Translations- und Rotationsbewegung des Antriebsglieds mitgenommene Kupplungseingangsglied stößt in der Maximaldosisposition des Antriebsglieds gegen einen Translationsanschlag des Kupplungsausgangsglieds. Durch weiteres Ziehen an dem Antriebsglied wird die Ratsche gelöst, und die Anordnung aus Antriebsglied und Kupplungseingangsglied kann zurückbewegt werden, um die korrekte Dosis einzustellen. Für die Korrektur muss das Antriebsglied wieder in den Ratscheneingriff mit dem Kupplungseingangsglied gebracht werden.

Muss bei dem Injektionsgerät der WO 01/10484 A1 die für die Ausschüttung in die Förder- und Dosiereinrichtung einzubringende Antriebskraft noch manuell aufgebracht werden, so geht aus der WO 02/053214 A1 eine Förder- und Dosiereinrichtung hervor, die für die Erzeugung der Antriebskraft eine Antriebsfeder verwendet, die durch das Einstellen der Dosis gespannt wird und die gespeicherte Energie bei der Ausschüttung freisetzt. Als Antriebsfeder dient eine spiralig um eine Achse gewundene Feder, deren Windungen entlang der Achse nebeneinander liegen. Wegen der Kopplung zwischen Antriebsfeder und Kolbenstange kann nur eine fest vorgegebene Dosis eingestellt werden. Die WO 2004/078240 A zeigt eine gattungsgemäße Vorrichtung.

Es ist eine Aufgabe der Erfindung, eine Vorrichtung zur Verabreichung eines fluiden Produkts zu schaffen, die einfach und sicher bedienbar ist und vorteilhafterweise Dosiskorrekturen einfach und sicher ermöglicht, und ungewollte Ausschüttbewegungen verhindert.

Gelöst wird die Aufgabe durch die Merkmale des Anspruchs 1

Die Erfindung betrifft eine Vorrichtung zur Verabreichung eines fluiden Produkts, die ein Gehäuse mit einem Reservoir aufweist. Das Reservoir kann selbst ein Behältnis für das Produkt bilden oder vorzugsweise ein Behältnis für das Produkt, beispielsweise eine Ampulle, aufnehmen. Die Vorrichtung umfasst ferner eine Fördereinrichtung für das Produkt, ein Antriebsglied und eine Kupplung für eine Kopplung und Entkopplung von Fördereinrichtung und Antriebsglied. Insbesondere können ein Kolben und eine Kolbenstange, die in eine Vortriebsrichtung auf den Kolben wirkt, die Fördereinrichtung bilden, wobei die Kolbenstange vorzugsweise lose gegen eine Rückseite des Kolbens drückt, grundsätzlich aber auch mit dem Kolben mittels einer auf Zug beanspruchbaren Verbindung verbunden oder einstückig mit dem Kolben gebildet sein kann.

Mittels des Antriebsglieds wird die Fördereinrichtung mit einer Antriebskraft beaufschlagt, um eine einstellbare Produktdosis auszuschütten. Das Antriebsglied wird durch die Einstellung der Produktdosis beeinflusst, indem es entweder selbst ein Dosierglied bildet, mittels dem die Produktdosis eingestellt werden kann, oder indem es durch eine Einstellung der Produktdosis in Abhängigkeit von der eingestellten Produktsdosis in eine bestimmte Position bewegt oder elastisch gespannt wird. Im letzteren Fall wird das Antriebsglied in Abhängigkeit von der eingestellten Produktdosis sozusagen mit Energie geladen, die es speichert und im Falle einer Auslösung freisetzt. Das Antriebsglied kann dementsprechend betätigbar sein, indem die Antriebskraft manuell über das Antriebsglied eingeleitet wird, oder es ist auslösbar und setzt im Falle der Auslösung die zuvor aufgenommene und gespeicherte Energie frei. Als auslösbares Antriebsglied muss es nicht unumgänglich durch die Einstellung der Dosis gespannt oder sonst wie aufgeladen werden, sondern kann beispielsweise auch als Elektromotor gebildet sein, der ausgelöst und durch einen entsprechend der Dosis eingestellten Schalter wieder ausgeschaltet wird.

Die Kupplung koppelt in einem Kupplungseingriff das Antriebsglied mit der Fördereinrichtung, um eine Antriebskraft des Antriebsglieds für eine Ausschüttung des Produkts auf die Fördereinrichtung zu übertragen. In einem ausgekuppelten Zustand entkoppelt sie die Fördereinrichtung von dem Antriebsglied, so dass Manipulationen an dem Antriebsglied oder auf das Antriebsglied wirkende Manipulationen, wie beispielsweise die Einstellung einer Dosis, insbesondere eine Dosiskorrektur, die Fördereinrichtung nicht beeinflussen können.

Die Kupplung besteht aus wenigstens zwei Kupplungsgliedern, von denen ein Kupplungseingangsglied mit dem Antriebsglied und ein Kupplungsausgangsglied mit der Fördereinrichtung gekoppelt sind. Wenigstens eines der Kupplungsglieder ist in den Kupplungseingriff bewegbar, und wenigstens eines der Kupplungsglieder, welches vorzugsweise das gleiche ist aber nicht sein muss, ist aus dem Kupplungseingriff bewegbar.

Nach der Erfindung umfasst die Vorrichtung ferner eine Halteeinrichtung, die die Kupplungsglieder in einer voneinander entkoppelten Halteposition hält. Dementsprechend ist wenigstens eines der Kupplungsglieder mit einer Kupplungsbewegung aus der Halteposition in den Kupplungseingriff bewegbar. In der Halteposition ist der Kraftfluss zwischen den Kupplungsgliedern unterbrochen. Im Kupplungseingriff hingegen ist die Antriebskraft des Antriebsglieds über die Kupplungsglieder auf die Fördereinrichtung übertragbar und bewirkt eine Ausschüttbewegung der Fördereinrichtung.

In der Halteposition sind die im Kupplungseingriff formschlüssig ineinander greifenden oder gegebenenfalls nur reibschlüssig gegeneinander gepressten Eingriffselemente der betreffenden Kupplungsglieder voneinander abgerückt. Die Kupplungsglieder selbst können einander auch in der Halteposition durchaus kontaktieren, ihre Eingriffselemente haben jedoch in der Halteposition keinen Kontakt. Bevorzugt sind jedoch die im eingekoppelten Zustand im Kupplungseingriff befindlichen Kupplungsglieder im ausgekuppelten·Zustand im Ganzen voneinander abgerückt.

Die Halteeinrichtung ist oder umfasst in bevorzugten Ausführungen ein Rückstellglied, das der Kupplungsbewegung mit einer elastischen Rückstellkraft entgegen wirkt. Anstatt die Kupplung, wie bevorzugt, mittels einer Elastizitätskraft im ausgekuppelten Zustand zu halten, könnte die Halteeinrichtung auch formschlüssig wirken, indem das wenigstens eine die Kupplungsbewegung ausführende Kupplungsglied am Gehäuse oder einer damit zumindest bezüglich der Kupplungsbewegung fest verbundenen Struktur durch einen lösbaren Formschluss fixiert wird.

Das Kupplungseingangsglied und das Kupplungsausgangsglied können im eingekuppelten Zustand der Kupplung unmittelbar miteinander in dem Kupplungseingriff sein. In einer Weiterbildung umfasst die Kupplung jedoch ein Kupplungszwischenglied, über das im Kupplungseingriff das Kupplungseingangsglied mit dem Kupplungsausgangsglied gekoppelt ist. In besonders bevorzugten Ausführungen wird der Kupplungseingriff zwischen dem Kupplungseingangsglied und dem Kupplungszwischenglied hergestellt. Das Kupplungszwischenglied ist vorzugsweise mit dem Kupplungsausgangsglied auch in der Halteposition der Kupplungsglieder, d.h. im ausgekuppelten Zustand, in einem Eingriff, in dem die Antriebskraft übertragbar ist. Vorteilhafterweise ist es im Eingriff relativ zu dem Kupplungsausgangsglied beweglich, vorzugsweise in und gegen die Richtung der Kupplungsbewegung.

Erfindungsgemäß wird in der Halteposition der Kupplungsglieder das Kupplungsausgangsglied an dem Gehäuse so festgelegt, dass es keine Bewegung ausführen kann, die eine Ausschüttbewegung der Fördereinrichtung bewirken würde. Das Kupplungsausgangsglied muss bewusst freigegeben werden, vorzugsweise unmittelbar im Zusammenhang mit der Ausschüttung des Produkts. Vorteilhaft ist es, wenn die Festlegung an dem Gehäuseteil durch Ausführung der Kupplungsbewegung gelöst wird. Vorzugsweise wird während eines bei der Kupplungsbewegung zurückgelegten Wegabschnitts in einer ersten Phase der Kupplungseingriff hergestellt und in einer sich anschließenden zweiten Phase die Festlegung an dem Gehäuse gelöst, vorteilhafterweise gegen die genannte elastische Rückstellkraft der Halteeinrichtung. Vorteilhafterweise ist das Kupplungsausgangsglied in der Halteposition der Kupplungsglieder über das Kupplungszwischenglied an dem Gehäuse festgelegt. Der Sperreingriff, in dem das Kupplungszwischenglied für diesen Zweck mit dem Gehäuse oder einer damit verbundenen Struktur steht, wird zweckmäßigerweise durch die Ausführung der Kupplungsbewegung gelöst. Vorteilhaft ist es, wenn das Kupplungszwischenglied in Richtung der Kupplungsbewegung aus dem Sperreingriff bewegbar ist, da eine derartige Bewegbarkeit es erlaubt, dass das Kupplungszwischenglied bei der Kupplungsbewegung einfach mitgenommen, beispielsweise aus dem Sperreingriff gedrückt wird. Der Sperreingriff kann formschlüssig und/oder reibschlüssig sein.

In einer ersten Variante wirkt das Rückstellglied über das Kupplungszwischenglied auf das wenigstens eine die Kupplungsbewegung ausführende Kupplungsglied und hält dieses in der Halteposition. In einer zweiten Variante wirkt das Rückstellglied direkt auf das wenigstens eine die Kupplungsbewegung ausführende Kupplungsglied, vorzugsweise das Kupplungseingangsglied, und stützt sich für die Beaufschlagung beispielsweise an dem Gehäuse oder einer damit bezüglich der Kupplungsbewegung fest mit dem Gehäuse verbundenen Struktur oder an dem Kupplungsausgangsglied ab.

In Ausführungen, in denen die Antriebskraft manuell aufgebracht wird, ist die mittels der Kupplungsglieder hergestellte Kopplung des Antriebsglieds und der Fördereinrichtung vorzugsweise so gebildet, dass die Antriebsbewegung des Antriebsglieds untersetzt wird. Die für einen Ausschüttvorgang einer bestimmten Dosis vom Antriebsglied zurückgelegte Wegstrecke, d.h. die Gesamtwegstrecke der Antriebsbewegung, ist in solchen Ausführungen länger als die Wegstrecke der hierdurch bewirkten Ausschüttbewegung der Fördereinrichtung.

Das Kupplungsausgangsglied ist mit der Fördereinrichtung in einem Eingriff, vorzugsweise einem Gewindeeingriff, kann grundsätzlich aber in alternativer Ausführung auch erst über Zwischenglieder mit der Fördereinrichtung gekoppelt sein, um die Ausschüttung zu bewirken. In dem bevorzugten Gewindeeingriff wird eine rotatorische Bewegung des Antriebsglieds in vorzugsweise eine translatorische Bewegung eines Förderglieds der Fördereinrichtung umgewandelt. Der Gewindeeingriff ist vorteilhafterweise nicht selbsthemmend, so dass durch eine in Richtung der Gewindeachse auf das im Gewindeeingriff translatorisch bewegbare Förderglied ausgeübte Kraft das Förderglied axial bewegbar ist.

Das Kupplungseingangsglied ist mit dem Antriebsglied vorzugsweise in einem Eingriff, kann grundsätzlich jedoch mit dem Antriebsglied alternativ auch über Zwischenglieder gekoppelt sein. Die Kopplung kann rein formschlüssig oder rein kraftschlüssig sein. Vorzugsweise ist sie form- und kraftschlüssig und besonders bevorzugt als Gewindeeingriff gebildet, insbesondere in der bevorzugten Ausführung, in der das Kupplungseingangsglied und das Antriebsglied miteinander in dem Eingriff sind. Der Gewindeeingriff ist vorteilhafterweise nicht selbsthemmend, so dass durch eine in Richtung der Gewindeachse auf das Antriebsglied wirkende Antriebskraft, das Antriebsglied im Gewindeeingriff axial bewegbar ist.

In Ausführungen, in denen das Kupplungseingangsglied in einem ersten Gewindeeingriff angetrieben wird, vorzugsweise rotatorisch, und das Kupplungsausgangsglied über einen weiteren, zweiten Gewindeeingriff oder vorzugsweise in einem zweiten Gewindeeingriff unmittelbar mit einem Förderglied dieses antreibt, vorzugsweise translatorisch, bilden die beiden Gewindeeingriffe ein Untersetzungsgetriebe, das die Wegstrecke der Antriebsbewegung in eine kürzere Wegstrecke der Ausschüttbewegung untersetzt. Die Untersetzung beträgt vorzugsweise wenigstens 2:1, noch bevorzugter wenigstens 3:1. Die Untersetzung kann vorteilhafterweise allein durch die unterschiedlichen Steigungen der beiden Gewindeeingriffe bzw. Spindeltriebe erreicht werden. So kann die Steigung im ersten Gewindeeingriff beispielsweise 60° und die Steigung im zweiten Gewindeeingriff 17° betragen.

Obgleich das Antriebsglied gleichzeitig auch ein Dosierglied der Vorrichtung bilden kann, an dem der Benutzer die Einstellung der Dosis vornimmt, umfasst die Vorrichtung in bevorzugten Ausführungen ein Dosierglied zusätzlich zu dem Antriebsglied. Das Dosierglied ist mit dem Antriebsglied so gekoppelt, vorzugsweise rein mechanisch, dass eine Dosierbewegung des Dosierglieds auch eine Dosierbewegung des Antriebsglieds zur Folge hat. Die Dosierbewegung des Antriebsglieds ist der von dem oder über das Antriebsglied bei der Ausschüttung aufgebrachten Antriebskraft vorzugsweise entgegengerichtet. Vorzugsweise verfügt die Vorrichtung über eine Dosisanzeige zum Anzeigen der eingestellten Produktdosis. Die Anzeige kann eine akustische und/oder taktile und/oder insbesondere optische sein. Die Dosisanzeige ist mit dem Dosierglied so gekoppelt, dass eine Bewegung, die das Dosierglied bei einer Einstellung der Produktdosis ausführt, eine Änderung der angezeigten Produktdosis bewirkt. In der Halteposition der Kupplungsglieder ist/sind das Dosierglied und/oder die Dosisanzeige von der Fördereinrichtung entkoppelt. Durch die Entkopplung kann die Dosis im ausgekuppelten Zustand ohne Rückwirkung auf die Fördereinrichtung eingestellt und gegebenenfalls korrigiert werden.

In bevorzugter Ausführung bleibt die Kopplung von Dosisanzeige und Antriebsglied im Kupplungseingriff bestehen, so dass eine der Dosierbewegung entgegengerichtete Antriebsbewegung des Antriebsglieds mit fortschreitender Ausschüttung in gleicher Weise fortschreitend zurückgestellt wird. Bei einem vorzeitigen Abbruch der Verabreichung, ob bewusst oder fehlerhaft unbewusst, zeigt die Dosisanzeige somit den noch nicht ausgeschütteten Rest der eingestellten Dosis an. Dies kann beispielsweise dann von Vorteil sein, wenn die eingestellte Dosis größer ist als die noch verfügbare.

Falls ein Dosierglied wie bevorzugt zusätzlich zu dem Antriebsglied vorgesehen ist, sind im Kupplungseingriff das Antriebsglied und das Dosierglied vorteilhafterweise voneinander entkoppelt, so dass während der Antriebsbewegung des Antriebsglieds an dem Dosierglied keine auf das Antriebsglied zurückwirkende Manipulationen vorgenommen werden können.

In Ausführungen, in denen das Antriebsglied das Kupplungseingangsglied wie bevorzugt rotatorisch antreibt, kann insbesondere eine Spiralfeder das Antriebsglied bilden. Die Spiralfeder ist um eine Rotationsachse der Rotationsbewegung gewunden, wobei wenigstens eine äußere Federwindung eine innere umgibt. Vorzugsweise weist die Feder bezüglich der Rotationsachse überall die Steigung Null auf. Durch die Verwendung der Spiralfeder kann axiale Baulänge eingespart werden, insbesondere im Vergleich zu den Federn aus dem Stand der Technik, deren Windungen axial nebeneinander angeordnet sind. Die Spiralfeder ist mit einem ihrer beiden Enden, vorzugsweise dem radial inneren Ende, verdrehgesichert mit dem Kupplungseingangsglied verbunden. Das andere Ende, vorzugsweise das radial äußere Ende, ist verdrehgesichert mit dem Gehäuse verbunden. Vorteilhafterweise bildet das Kupplungseingangsglied eine Trommel, auf der die Spiralfeder aufgewickelt ist. Bei der Einstellung der Dosis wird das Kupplungseingangsglied um die Rotationsachse gedreht, wodurch die Spiralfeder gespannt wird. Durch eine geeignete Verdrehsperre, beispielsweise eine Ratsche, wird sichergestellt, dass das Kupplungseingangsglied nur in eine Richtung gedreht werden kann. Die Verdrehsperre ist vorzugsweise lösbar, um eine falsch eingestellte Dosis korrigieren zu können. Bei einem Lösen der Verdrehsperre kann sich bei fehlerhafter Bedienung schlimmstenfalls das Kupplungseingangsglied unter der Einwirkung der gespannten Antriebs feder zu weit zurückdrehen. Da der Kupplungseingriff, der das Kupplungseingangsglied mit dem Kupplungsausgangsglied koppelt, bei der Einstellung der Dosis noch nicht hergestellt ist, da die Kupplungsglieder in der Halteposition gehalten werden, können derartige Fehlbedienungen sich nicht auf die Fördereinrichtung auswirken.

Die Kupplungsbewegung ist vorzugsweise eine axiale Hubbewegung. Falls ein Kolben und eine Kolbenstange die Fördereinrichtung bilden, wird die Hubbewegung bevorzugt in Vortriebsrichtung des Kolbens und der Kolbenstange ausgeführt. Treibt die Antriebskraft das Kupplungseingangsglied und dieses über den Kupplungseingriff das Kupplungsausgangsglied rotatorisch an, vorteilhafterweise um eine in die Vortriebsrichtung weisende Rotationsachse, so können diejenigen Kupplungsglieder, die zwischen sich den Kupplungseingriff bilden, insbesondere mit Eingriffselementen versehen sein, die im Kupplungseingriff wie axial zueinander verschiebbare Nuten und Federn zusammenwirken oder als axial einander zugewandte Verzahnungen oder bevorzugter als Kegelverzahnungen gebildet sein. Obgleich beispielsweise ein einziger Zahn und eine einzige Zahnlücke für den Kupplungseingriff grundsätzlich genügen, wird es bevorzugt, wenn bei wenigstens einem der den Kupplungseingriff bildenden Kupplungsglieder eine Verzahnung um die Rotationsachse umlaufend gebildet ist. Noch bevorzugter weisen die beiden Kupplungsglieder für den Kupplungseingriff je eine Verzahnung umlaufend auf. Bei als Nuten und Federn oder andersartig gebildeten Eingriffselementen gilt entsprechendes. Der Kupplungseingriff ist ungeachtet der Frage der Form der Kupplungsflächen so gebildet, das Schlupf im Kupplungseingriff nicht auftritt.

Um ein schlankes, kompaktes Injektionsgerät zu erhalten, sollten die Kolbenstange und das Antriebsglied oder sollten die Kolbenstange und das Kupplungseingangsglied, bevorzugt die Kolbenstange, das Antriebsglied und das Kupplungseingangsglied, zueinander koaxial angeordnet sein. Eines dieser Glieder sollte wenigstens ein anderes umgeben, bevorzugt sind alle drei Glieder geschachtelt angeordnet. Desweiteren ist es vorteilhaft, wenn das Kupplungsausgangsglied ebenfalls koaxial zu der Kolbenstange und/oder dem Kupplungseingangsglied angeordnet ist, wobei es insbesondere die Kolbenstange umgeben oder gegebenenfalls von der Kolbenstange umgeben sein kann.

Bevorzugte Merkmale werden auch in den Unteransprüchen und deren Kombinationen beschrieben.

Obgleich die vorstehend beschriebenen Weiterbildungen und die in den Unteransprüchen beschriebenen Merkmale besonders vorteilhaft den Gegenstand von Anspruch 1 weiterbilden, sind sie vorteilhaft auch für Injektionsgeräte, die zwar die erfindungsgemäße Kupplung, nicht aber unbedingt das Merkmal der Halteeinrichtung und des Haltens in der Halteposition aufweisen.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausführungen vorteilhaft weiter. Es zeigen:
- Figur 1: ein Injektionsgerät eines ersten Ausführungsbeispiels in einer perspektivischen Ansicht,
- Figur 2: das Injektionsgerät mit einer geöffneten Kupplung in einem Längsschnitt,
- Figur 3: das Injektionsgerät mit geschlossener Kupplung,
- Figur 4: ein Detail der Figur 2,
- Figur 5: ein Detail der Figur 3,
- Figur 6: das Injektionsgerät nach Einstellung einer Dosis,
- Figur 7: das Injektionsgerät nach Entleerung eines Reservoirs,
- Figur 8: ein Entkopplungsglied und ein Gehäuseteil des Injektionsgeräts,
- Figur 9: einen distalen Abschnitt des Injektionsgeräts mit verbundenen Gehäuseteilen,
- Figur 10: den distalen Abschnitt während des Lösens der Gehäuseteile
- Figur 11: ein Injektionsgerät eines zweiten Ausführungsbeispiels mit geöffneter Kupplung in einem Längsschnitt,
- Figur 12: das Injektionsgerät des zweiten Ausführungsbeispiels mit geschlossener Kupplung in einem anderen Längsschnitt,
- Figur 13: ein Detail der Figur 11,
- Figur 14: ein Detail der Figur 12,
- Figur 15: das Injektionsgerät des zweiten Ausführungsbeispiels nach Einstellung einer Dosis,
- Figur 16: das Injektionsgerät des zweiten Ausführungsbeispiels nach Entleerung des Reservoirs,
- Figur 17: das Injektionsgerät des zweiten Ausführungsbeispiels mit voneinander gelösten Gehäuseteilen,
- Figur 18: ein Detail der Figur 17,
- Figur 19: ein Injektionsgerät eines dritten Ausführungsbeispiels
- Figur 20: einen proximalen Teil des Injektionsgeräts des dritten Ausführungsbeispiels mit geöffneter Kupplung,
- Figur 21: das Injektionsgerät des dritten Ausführungsbeispiels mit geschlossener Kupplung,
- Figur 22: den proximalen Teil des Injektionsgeräts des dritten Ausführungsbeispiels bei einer Dosiskorrektur,
- Figur 23: den proximalen Teil des Injektionsgeräts des dritten Ausführungsbeispiels nach Entleerung des Reservoirs,
- Figur 24: ein Sperrglied und ein Stoppglied des dritten Ausführungsbeispiels.

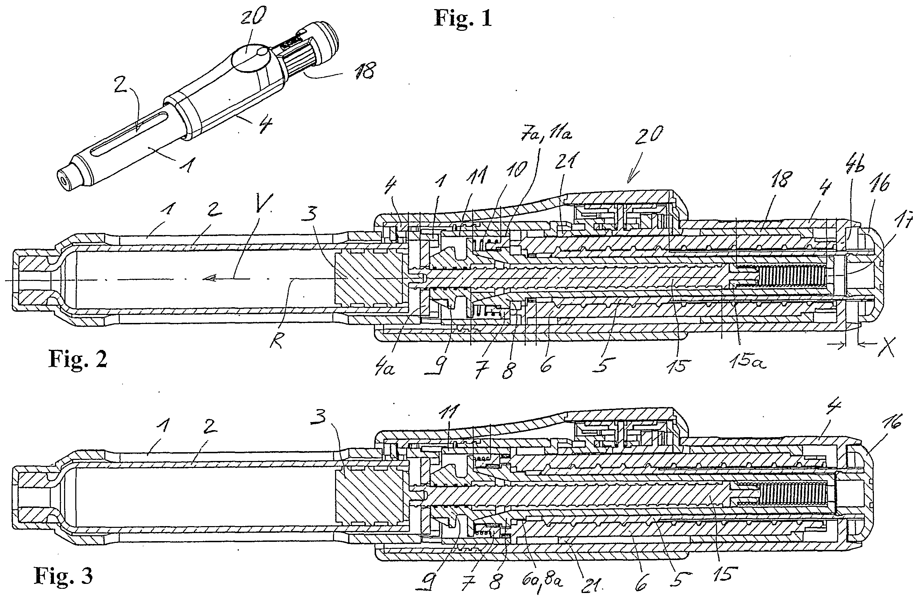

Figur 1 zeigt ein Injektionsgerät eines ersten Ausführungsbeispiels. Das Injektionsgerät weist ein erstes Gehäuseteil 1 und ein zweites Gehäuseteil 4 auf, die lösbar miteinander verbunden sind. Im Ausführungsbeispiel sind die Gehäuseteile 1 und 4 miteinander verschraubt. Das Injektionsgerät ist als schlanker Injektionspen gebildet. Das Gehäuseteil 1 dient der Aufnahme eines mit fluidem Produkt gefüllten Behältnisses 2 und bildet in diesem Sinne ein Reservoir, und das Gehäuseteil 4 dient als Träger für eine Dosier- und Antriebseinrichtung, von der ein Dosierglied 18 zu erkennen ist. Das Gehäuseteil 4 ist im Bereich des Dosierglieds 18 durchbrochen, so dass ein Verwender direkten Zugriff auf das Dosierglied 18 hat. Das Dosierglied 18 ist um eine zentrale Längsachse des Geräts drehbar gelagert und als Hülse gebildet, die bedienerfreundlich an ihrem äußeren Umfang geriffelt ist. Zu erkennen ist ferner eine Dosisanzeige 20, die durch eine Durchbrechung im Mantel des Gehäuseteils 4 seitlich aufgesetzt ist.

Figur 2 zeigt das Injektionsgerät des ersten Ausführungsbeispiels in einem Längsschnitt. In dem Gehäuseteil 1 ist das Behältnis 2 aufgenommen. In dem Behältnis 2 ist ein Kolben 3 in eine Vortriebsrichtung V bewegbar aufgenommen. Der Kolben 3 verschließt das Behältnis 2 an dessen proximalen Ende flüssigkeitsdicht. Durch Vortrieb des Kolbens 3 in die Vortriebsrichtung V wird Produkt durch einen Auslass des Behältnisses 2 verdrängt und ausgeschüttet, vorzugsweise durch eine in den Auslass ragende, mittels eines Nadelhalters an dem distalen Ende des Gehäuseteils 1 befestigten Injektionsnadel. Das Behältnis 2 ist in der Art üblicher Ampullen gebildet. Das Gehäuseteil 1 bildet unmittelbar einen Behältnishalter, im Ausführungsbeispiel einen Ampullenhalter. Das Gehäuseteil 1 ragt mit seinem proximalen Ende in das Gehäuseteil 4 hinein, und ist mit dem Gehäuseteil 4 verschraubt.

Das Gehäuseteil 4 nimmt eine Kolbenstange 15 und die Dosier- und Antriebseinrichtung auf, die als Dosier- und Antriebsmechanik gebildet ist. Die Dosier- und Antriebseinrichtung umfasst in einem Dosier- und Antriebsstrang ein Antriebsglied 5 und eine Kupplung 6-11, die in einem eingekuppelten Zustand, d.h. in einem Kupplungseingriff, das Antriebsglied 5 mit der Kolbenstange 15 koppelt. Die Kolbenstange 15 bildet mit dem Kolben 3 eine Fördereinrichtung. Im eingekuppelten Zustand übertragen Kupplungsglieder 6-10 eine auf das Antriebsglied 5 ausgeübte Antriebskraft auf die Kolbenstange 15. In Figur 2 besteht kein Kupplungseingriff, so dass die Kolbenstange 15 von dem Antriebsglied 5 entkoppelt ist. In diesem ausgekuppelten Zustand kann der Benutzer durch eine Dosierbewegung des Dosierglieds 18, im Ausführungsbeispiel eine Drehbewegung, die zu verabreichende Produktdosis einstellen.

Das Antriebsglied 5 ist hülsenförmig. Es weist an seiner Mantelaußenfläche ein Gewinde um eine in die Vortriebsrichtung V weisende Gewindeachse R auf. Mit diesem Gewinde steht das Antriebsglied 5 in einem Gewindeeingriff mit einem Kupplungseingangsglied 6. Das Kupplungseingangsglied 6 ist ebenfalls hülsenförmig und für den Gewindeeingriff mit einem korrespondierenden Innengewinde versehen. Die Gewindesteigung im Gewindeeingriff ist so groß, dass eine Selbsthemmung nicht eintreten kann. Das Dosierglied 18 umgibt das Kupplungseingangsglied 6 und ist mit dem Kupplungseingangsglied 6 verdrehgesichert und axial nicht beweglich verbunden. Die Kolbenstange 15 ragt in das Antriebsglied 5 und das Kupplungseingangsglied 6.

Die Kolbenstange 15 ist über ihre axiale Länge mit einem Außengewinde versehen. Mit dem Außengewinde ist sie in einem Gewindeeingriff mit einem Kupplungsausgangsglied 9, das mit einem korrespondierenden Innengewinde versehen ist. Auch diese beiden Gewinde weisen eine Steigung auf, die eine Selbsthemmung im Gewindeeingriff verhindert. Die Gewindesteigung ist vorzugsweise geringer als die Gewindesteigung im Gewindeeingriff zwischen dem Antriebsglied 5 und dem Kupplungseingangsglied 6. Eine Kupplungshülse 8 ist mit dem Kupplungsausgangsglied 9 verdrehgesichert und axial nicht beweglich verbunden. Die Kupplungshülse 8 und das Kupplungsausgangsglied 9 können bezüglich der zwischen dem Antriebsglied 5 und der Kolbenstange 15 stattfindenden Bewegungen wie ein einstückiges Bauteil angesehen werden; sie sind zur Aufnahme einer Ausgleichsfeder 17 jedoch zweiteilig ausgeführt und fest miteinander verbunden. Das Kupplungsausgangsglied 9 und die Kupplungshülse 8 sind um die Gewindeachse R des Kupplungsausgangsglieds 9 drehbar, aber axial nicht bewegbar im Gehäuseteil 4 gelagert. Die Kolbenstange 15 durchragt im Gewindeeingriff des Kupplungsausgangsglied 9 und ragt in die Kupplungshülse 8 hinein. Zwischen einem proximalen Ende der Kupplungshülse 8 und einem proximalen Ende der Kolbenstange 15 ist die Ausgleichsfeder 17 eingespannt, die als Druckfeder in Vortriebsrichtung V auf die Kolbenstange 15 wirkt. Die Ausgleichsfeder 17 drückt über einen drehbar an der Kolbenstange 15 abgestützten Teller 15a, den ein Flansch einer auf die Kolbenstange 15 gesetzten Hülse bildet, auf die Kolbenstange 15.

Die Kolbenstange 15 ist in einer Linearführung 4a relativ zu dem Gehäuseteil 1 nicht verdrehbar in und gegen die Vortriebsrichtung V linear geführt. Das Antriebsglied 5 ist relativ zu dem Gehäuseteil 4 ebenfalls in und gegen die Vortriebsrichtung V bewegbar lineargeführt, wofür das Gehäuseteil 4 unmittelbar eine Linearführung 4b bildet.

Die Gewindeachse der Kolbenstange 15 bildet die Hauptbewegungsachse der Vorrichtung. Für die rotatorische Antriebsbewegung des Kupplungseingangsglieds 6 und über das Kupplungszwischenglied 7 auch des Kupplungsausgangsglieds 9 bildet sie eine Rotationsachse R. Sie bildet beide Gewindeachsen. Desweiteren bildet sie die Translationsachse für die Kolbenstange 15 und das Antriebsglied 5.

Die Kupplung 6-11 umfasst ferner ein Kupplungszwischenglied 7 und ein Rückstellglied 10, das als Druckfeder gebildet ist, und das Kupplungszwischenglied 7 mit einer gegen die Vortriebsrichtung V wirkenden Elastizitätskraft beaufschlagt. Das Rückstellglied 10 ist zwischen dem Kupplungsausgangsglied 9 und dem Kupplungszwischenglied 7 eingespannt.

Falls auf das Antriebsglied 5 keine in die Vortriebsrichtung V wirkende Kraft ausgeübt wird, sorgt das Rückstellglied 10 über das Kupplungszwischenglied 7 dafür, dass der Kupplungseingriff gelöst ist. Diesen Zustand zeigt die Figur 2. Das Kupplungseingangsglied 6 ist in die Vortriebsrichtung V auf Anschlag gegen das Kupplungszwischenglied 7 und wird von dem Rückstellglied 10 über das Kupplungszwischenglied 7 in eine proximale Endposition gedrückt. Das Rückstellglied 10 hält das Kupplungseingangsglied 6 relativ zu dem Kupplungsausgangsglied 9 und der daran befestigten Kupplungshülse 8 mittels des Kupplungszwischenglieds in einer Halteposition. Das Rückstellglied 10 und das Kupplungszwischenglied 7 bilden somit eine kraftschlüssig wirkende Halteeinrichtung für das Kupplungseingangsglied 6.

Figur 3 zeigt das Injektionsgerät in einem gekoppelten Zustand. Zwischen dem Kupplungseingangsglied 6 und der Kupplungshülse 8 besteht ein Kupplungseingriff. Für den Kupplungseingriff bilden das Kupplungseingangsglied 6 und die Kupplungshülse 8 Eingriffselemente, die im Kupplungseingriff eine verdrehgesicherte Verbindung der beiden Glieder 6 und 8 um die in die Vortriebsrichtung V weisende Gewindeachse R herstellen. Die Eingriffselemente wirken als Nuten und Federn oder Verzahnungen zusammen, die zur Vortriebsrichtung V parallel und um die Gewindeachse R gleichmäßig verteilt gebildet sind.

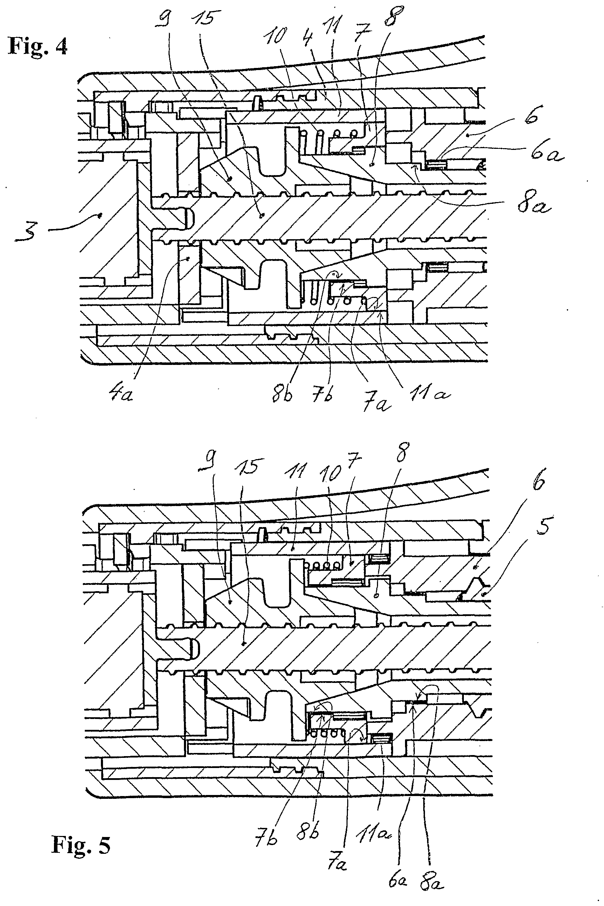

Die Figuren 4 und 5 zeigen den Bereich des Kupplungseingriffs im Detail. Figur 4 zeigt das Gerät in dem entkoppelten und Figur 5 in dem gekoppelten Zustand. Figur 4 korrespondiert somit mit Figur 2, und Figur 5 korrespondiert mit Figur 3.

Im entkoppelten Zustand ist das Kupplungseingangsglied 6 entgegen der Vortriebsrichtung V von der Kupplungshülse 8 abgerückt, so dass das Kupplungseingangsglied 6 relativ zu der Kupplungshülse 8 und dem damit fest verbundenen Kupplungsausgangsglied 9 frei drehbar ist. Gleichzeitig ist das Kupplungsausgangsglied 9 über die Kupplungshülse 8, das Kupplungszwischenglied 7 und ein Entkopplungsglied 11 verdrehgesichert mit dem Gehäuseteil 4 verbunden. Für diese verdrehsichere Kopplung sind das Kupplungszwischenglied 7 an einer der Kupplungshülse 8 radial zugewandten Innenfläche mit Eingriffselementen 7b und die Kupplungshülse 8 mit korrespondierenden Eingriffselementen 8b versehen. Für den verdrehgesicherten Eingriff mit dem Entkopplungsglied 11 sind das Kupplungszwischenglied 7 an einer äußeren Umfangsfläche mit Eingriffselementen 7a und das Entkopplungsglied 11 an einer Mantelinnenfläche mit radial zugewandten Eingriffselementen 11a versehen, die im entkoppelten Zustand wie die Eingriffselemente 7b und 8b in der Art von Nuten und Federn oder Verzahnungen, die zur Vortriebsrichtung V parallel sind, ineinander greifen. Das Kupplungszwischenglied 7 ist in seinem verdrehgesicherten Eingriff mit der Kupplungshülse 8 und seinem verdrehgesicherten Eingriff mit dem Entkopplungsglied 11 in und gegen die Vortriebsrichtung V axial bewegbar, wobei sich der Eingriff mit dem Entkopplungsglied 11 bei einer Bewegung in die Vortriebsrichtung V löst.

Wird das Antriebsglied 5 betätigt, indem auf ein Auslöseelement 16 in die Vortriebsrichtung V eine Druckkraft ausgeübt wird, vollführen das Antriebsglied 5 und das Kupplungseingangsglied 6 gemeinsam einen axialen Kupplungshub der Länge X. Bei dieser Antriebshubbewegung bzw. Kupplungsbewegung stößt das Kupplungseingangsglied 6 das Kupplungszwischenglied 7 gegen die rückstellende Elastizitätskraft des Rückstellglieds 10 in die Vortriebsrichtung V. Im Verlaufe der Hubbewegung gelangen die Eingriffselemente 6a und 8a miteinander in Eingriff, während das Kupplungszwischenglied 7 sich gleichzeitig relativ zu dem Entkopplungsglied 11 bewegt bis es aus dem verdrehgesicherten Eingriff mit dem Entkopplungsglied 11 gelangt. Dabei bleibt das Kupplungszwischenglied 7 in dem verdrehgesicherten Eingriff mit der Kupplungshülse 8. Die Kupplungsbewegung wird durch einen Anschlag des Auslöseelements 16 an der Kupplungshülse 8, im Ausführungsbeispiel an deren proximalen Stirnfläche, begrenzt (Figur 3).

Figur 5 zeigt das Injektionsgerät im gekoppelten Zustand. Die Eingriffselemente 6a und 8a sind in axialer Überdeckung, so dass der Kupplungseingriff als verdrehgesicherter Eingriff zwischen dem Kupplungseingangsglied 6 und der Kupplungshülse 8 hergestellt ist. Der Eingriff zwischen dem Kupplungszwischenglied 7 und dem Entkopplungsglied 11 löst sich erst, nachdem der Kupplungseingriff sicher hergestellt ist.

Für die Einstellung der Dosis verdreht der Verwender das Dosierglied 18, das dabei in leicht lösbaren Rastpositionen verrastet. Das Dosierglied 18 ist verdrehgesichert und auch axial unbeweglich mit dem Kupplungseingangsglied 6 verbunden, so dass dieses mitdreht. Durch diese Dosierbewegung des Kupplungseingangsglieds 6 wird das bei 4b in und gegen die Vortriebsrichtung V linear geführte Antriebsglied 5 in die proximale Richtung bewegt und ragt dann aus dem Gehäuseteil 4. Der axiale Dosierweg des Antriebsglieds 5 ergibt sich aus dem Drehwinkel, um den das Dosierglied 18 verdreht wird und der Gewindesteigung im Gewindeeingriff zwischen dem Antriebsglied 5 und dem Kupplungseingangsglied 6, das in die Vortriebsrichtung V gegen das Kupplungszwischenglied 7 und gegen die Vortriebsrichtung V gegen das Gehäuseteil 4 auf Anschlag ist.

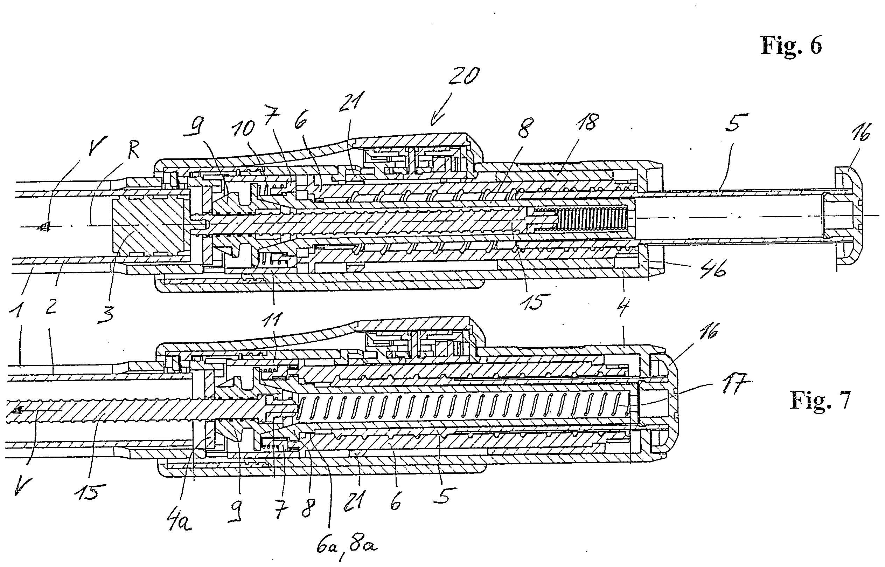

Figur 6 zeigt das Injektionsgerät mit noch vollständig gefülltem Behältnis 2 nach dem Einstellen einer ersten Dosis. In diesem Zustand durchsticht der Benutzer mit der Injektionsnadel die Haut für eine subkutane Injektion. Nachdem die Injektionsnadel platziert ist, betätigt der Benutzer das Antriebsglied 5, indem er dieses in die Vortriebsrichtung V in das Gehäuseteil 4 hinein drückt. In dem ersten Abschnitt der Antriebsbewegung, der Kupplungsbewegung bzw. dem Kupplungshub X, nimmt das Antriebsglied 5 das Kupplungseingangsglied 6 gegen die elastische Rückstellkraft des Rückstelldements 10 mit bis der Kupplungseingriff mit der Kupplungshülse 8 hergestellt und der verdrehgesicherte Eingriff zwischen dem Kupplungszwischenglied 7 und dem Entkopplungsglied 11 gelöst sind. Sobald die Kupplungshülse 8 und damit gemeinsam das Kupplungsausgangsglied 9 um die gemeinsame Rotationsachse R frei drehen können, ist der Kupplungshub X beendet, und es schließt sich als zweiter Abschnitt der Antriebsbewegung ein Ausschütthub an. Während des Ausschütthubs wird das Antriebsglied 5 weiter in die Vortriebsrichtung V gedrückt. Da das Kupplungseingangsglied 6 über den axialen Anschlag gegen das Kupplungszwischenglied 7 keine weitere Bewegung in die Vortriebsrichtung V ausführen kann, dreht es sich in dem Gewindeeingriff mit dem verdrehgesichert geführten Antriebsglied 5 um die gemeinsame Gewindeachse R. Das Kupplungseingangsglied 6 nimmt bei seiner Drehbewegung im Kupplungseingriff die Kupplungshülse 8 und diese das Kupplungsausgangsglied 9 mit. Die Kupplungshülse 8 ist gemeinsam mit dem Kupplungsausgangsglied 9 axial nicht bewegbar in dem Gehäuseteil 4 gehalten. Die Drehbewegung des Kupplungsausgangsglieds 9 bewirkt über den Gewindeeingriff mit der Kolbenstange 15 und deren verdrehgesicherten Linearführung bei 4a den Vortrieb und somit die Ausschüttbewegung der Kolbenstange 15 und damit gemeinsam des Kolbens 3. Sobald der Injektionsknopf 16 im Zuge der Antriebs-und Ausschüttbewegung in Anschlagkontakt gegen die Kupplungshülse 8 gelangt (Figur 3), ist der Ausschüttvorgang beendet.

Nimmt der Benutzer den Druck von dem Auslöseelement 16, so bewegt das Rückstellglied 10 über das Kupplungszwischenglied 7 das Kupplungseingangsglied 6 wieder in die aus dem Kupplungseingriff abgerückte Halteposition, wie sie in den Figuren 2 und 4 dargestellt ist. Das Kupplungseingangsglied 6 und damit gemeinsam das Antriebsglied 5, das Dosierglied 18 und die Dosisanzeige 20 werden durch die Ausrückbewegung des Kupplungseingangsglieds 6 von dem Kupplungsausgangsglied 9 und somit von der Kolbenstange 15 entkoppelt. Andererseits wird die Kolbenstange 15 über das zurückfahrende Kupplungszwischenglied 7 und das Entkopplungsglied 11 wieder verdrehgesichert mit dem Gehäuseteil 4 verbunden.

Figur 7 zeigt das Injektionsgerät am Ende einer letzten Ausschüttung, mit der das Behältnis 2 entleert wurde.

Für einen Austausch des entleerten Behältnisses 2 wird das Gehäuseteil 1 von dem Gehäuseteil 4 gelöst, im Ausführungsbeispiel durch eine Schraubbewegung. Bei dem Lösen der Gehäuseteile 1 und 2 wird das Entkopplungsglied 11 automatisch relativ zu dem Gehäuseteil 4 gegen die Richtung der Kupplungsbewegung des Kupplungseingangsglieds 6, im Ausführungsbeispiel gegen die Vortriebsrichtung V, bewegt. Das Gehäuseteil 4 lagert das Entkopplungsglied 11 entsprechend. Der von dem Entkopplungsglied 11 relativ zu dem Gehäuseteil 4 dabei zurückgelegte axiale Weg ist so lang wie der Kupplungshub X, so dass das Entkopplungsglied 11 nach dem Lösen der Gehäuseteile 1 und 4 dem Kupplungseingangsglied 6 axial gegenüberliegend dieses blockiert und das Kupplungseingangsglied 6 nicht mehr in die Vortriebsrichtung V bewegt werden kann, zumindest nicht in den Kupplungseingriff mit der Kupplungshülse 8. Die Blockierung des Kupplungseingangsglieds 6 in der ausgerückten Position verhindert, dass das Kupplungsausgangsglied 9 in eine verdrehgesicherte Verbindung mit dem Gehäuseteil 4 gelangen und dadurch ein Zurückschieben der Kolbenstange 15 verhindem kann. Es wird mit anderen Worten sichergestellt, dass die Kolbenstange 15 blockierfrei in das Gehäuseteil 4 zurückgeschoben werden kann.

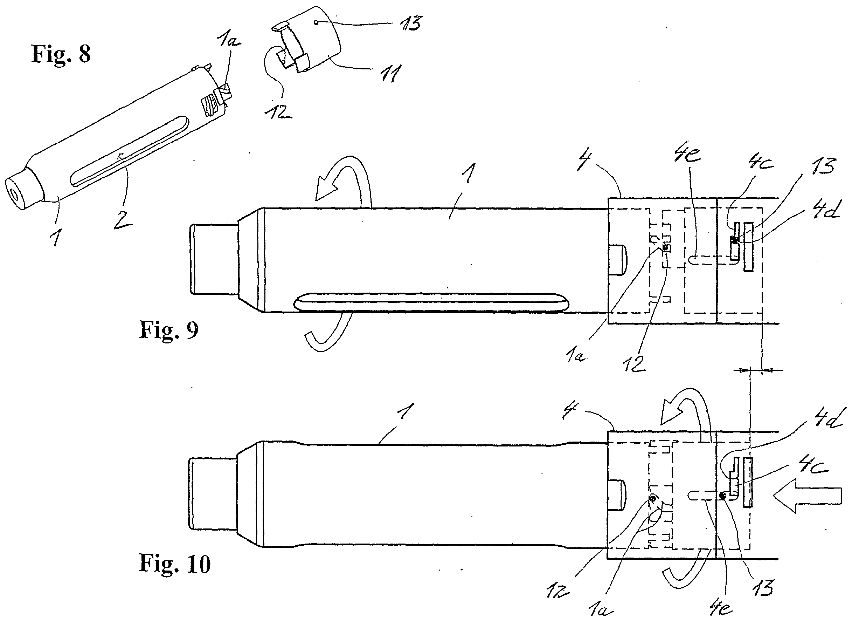

Figur 8 zeigt das Entkopplungsglied 11 und das erste Gehäuseteil 1 in einer perspektivischen Ansicht. Das Entkopplungsglied 11 ist ein Hülsenteil und weist in einem distalen Abschnitt drei nach radial einwärts ragende Eingriffselemente 12 und in einem proximalen Abschnitt ein nach radial auswärts ragendes Fixierelement 13 auf.

Figur 9 zeigt das Gehäuseteil 1 und einen Verbindungsabschnitt des Gehäuseteils 4, wobei das verdeckte Entkopplungsglied 11 strichliert dargestellt ist. Für seine Entkopplungsfunktion ist das Entkopplungsglied 11 in dem Verbindungsabschnitt des Gehäuseteils 4 drehbar und axial bewegbar aufgenommen. Die relative Bewegbarkeit wird durch eine Axialführung 4e und eine Umfangsführung 4c bestimmt, entlang denen das Eingriffselement 13 nacheinander entlangfährt, wenn das Gehäuseteil 1 von dem Gehäuseteil 4 gelöst wird. Die Umfangsführung 4c erstreckt sich rechtwinklig zur Axialführung 4e in Umfangsrichtung um die Schraubachse. Sie ist als Durchbruch oder Ausnehmung im Gehäuseteil 4 gebildet.

Mit dem Gehäuseteil 1 ist das Entkopplungsglied 11 in einem Führungseingriff. Für den Führungseingriff ist an einer Mantelaußenfläche des Gehäuseteils 1 pro Eingriffselement 12 eine Führungskurve 1a gebildet, die das Eingriffselement 12 und somit das Entkopplungsglied 11 bei dem Lösen der Gehäuseteile 1 und 4 führt. Eine parallel beabstandete weitere Führungskurve 1a führt das Entkopplungsglied 11 bei einem Verbinden der Gehäuseteile 1 und 4 entsprechend (Fig. 10). Die Führungskurve 1a verläuft in einem distalen Abschnitt schräg, d.h. mit einer Steigung, zu der Schraubachse der Schraubverbindung der Gehäuseteile 1 und 4, so dass bei der für das Lösen erforderlichen Relativdrehung zwischen den Gehäuseteilen 1 und 4 das Eingriffselement 12 an der Führungskurve 1a entlang gleitend eine Axialbewegung des Entkopplungsglieds 11 relativ zu dem Gehäuseteil 4 gegen die Vortriebsrichtung V ausführt, bis das Fixierelement 13 auf die axiale Höhe der Umfangsführung 4c gelangt. Die Steigung beträgt etwa 45° und ist konstant. Grundsätzlich kann sie aus dem gesamten Bereich größer 0° und kleiner 180° gewählt werden und gegebenenfalls auch veränderlich sein, solange die zum Lösen der Gehäuseteile 1 und 4 erforderliche Relativbewegung, im Ausführungsbeispiel eine Schraubbewegung, eine Bewegung des Entkopplungsglieds entgegen der vom Kupplungseingangsglied zum Einkuppeln auszuführenden Kupplungsbewegung X bewirkt. Ein distaler Abschnitt der Führungskurve 1a verläuft axial, so dass bei dem weiteren Auseinanderschrauben der Gehäuseteile 1 und 4 das Fixierelement 13 längs der Umfangsführung 4c bewegt wird. Im Verlaufe dieser relativen Umfangsbewegung zwischen dem Entkopplungsglied 11 und dem Gehäuseteil 4 gleitet das Fixierelement 13 über ein Fixierelement 4d im Bereich der Umfangsführung 4c. Das Fixierelement 4d ist als Nocken an einem Streifenabschnitt des Gehäuseteils 4 gebildet. Der Streifenabschnitt wirkt als beidseits fest eingespannte Biegefeder, die bei der Bewegung des Fixierelements 13 über das Fixierelement 4d federnd nachgibt, um anschließend wieder in ihre Ausgangslage zurückzufedern und einen lösbaren Rasteingriff für das Entkopplungsglied 11 zu bilden. In der Rastposition ist das Fixierelement 13 in die eine Umfangsrichtung an dem Fixierelement 4d und in die andere Umfangsrichtung an einer in der Umfangsführung 4c gebildeten Schulter auf Anschlag und dadurch in beide Umfangsrichtungen fixiert.

Figur 10 zeigt die beiden Gehäuseteile 1 und 4 und das Entkopplungsglied 11 nachdem dessen Fixierelement 13 hinter das Fixierelement 4d des Gehäuseteils 4 bewegt worden ist. Das Entkopplungsglied 11 ist über die Fixierelemente 4d und 13 mit dem Gehäuseteil 4 in dem lösbaren Rasteingriff und auf diese Weise an dem Gehäuseteil 4 verdrehgesichert und axial fixiert. In der in Figur 10 gezeigten Rastposition blockiert das Entkopplungsglied 11 das Kupplungseingangsglied 6 und stellt dadurch die Entkopplung von Antriebsglied 5 und Kolbenstange 15 sicher. Sobald das Entkopplungsglied 11 die Rastposition erreicht hat, bewegt sich dessen Eingriffselement 12 bei dem weiteren Auseinanderschrauben der Gehäuseteile 1 und 4 aus dem Führungseingriff mit der Führungskurve 1a. Die Führungskurve 1a ist entsprechend geformt.

Bei einem erneuten Zusammenschrauben der Gehäuseteile 1 und 4 zentrieren sich diese bezüglich der Umfangsrichtung mittels zusammenwirkender Zentrierelemente, so dass das Eingriffselement 12 des Entkopplungsglieds 11 wieder in Eingriff mit der Führungskurve 1a gelangt. Sobald der Führungseingriff hergestellt ist wird bei dem weiteren Zusammenschrauben das Entkopplungsglied 11 automatisch aus dem Rasteingriff der Fixierelemente 4d und 13 bewegt, bis es relativ zu dem Gehäuseteil 4 wieder die gleiche Position wie in Figur 9 und den Figuren 2 bis 7 einnimmt; dies entspricht der Betriebsposition des Entkopplungsglieds 11.

Bei oder vor dem Zusammenschrauben wird die Kolbenstange 15 einfach in das Gehäuseteil 4 zurückgeschoben, was wegen des gelösten Kupplungseingriffs eine Drehbewegung des Kupplungsausgangsglieds 9 bewirkt.

Die Dosisanzeige 20 des ersten Ausführungsbeispiels ist über ein Anzeigekopplungsglied 21 und das Kupplungseingangsglied 6 mit dem Antriebsglied 5 gekoppelt. Das Anzeigekopplungsglied 21 ist mit dem Kupplungseingangsglied 6 verdrehgesichert verbunden, indem es im Ausführungsbeispiel einen Ring bildend auf dem Kupplungsglied 6 und relativ zu diesem in und gegen die Richtung der Kupplungsbewegung X bewegbar ist. Das Anzeigekopplungsglied 21 ist umgekehrt zu dem Gehäuseteil 4 um die Rotationsachse R drehbar, wird relativ zu dem Gehäuseteil 4 jedoch axial nicht bewegbar gehalten. Das Anzeigekopplungsglied 21 weist umlaufend eine Verzahnung auf, die im Ausführungsbeispiel als Kegelverzahnung gebildet ist, mit der es mit einem Getriebe der Dosisanzeige 20 in Zahneingriff ist, um die Dosierbewegung und auch die Antriebsbewegung in das Getriebe einzuleiten.

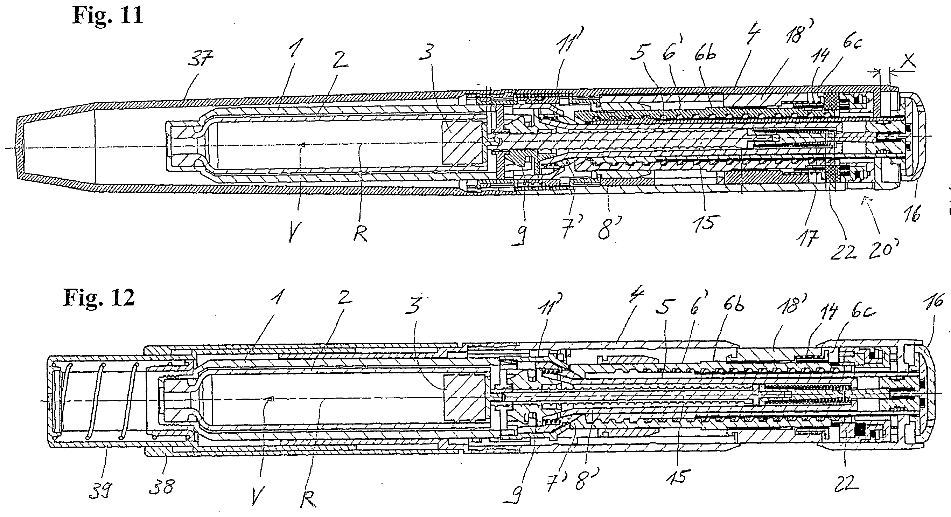

Die Figuren 11 bis 18 zeigen ein Injektionsgerät eines zweiten Ausführungsbeispiels. Das Injektionsgerät des zweiten Ausführungsbeispiels weist gegenüber dem Gerät des ersten Ausführungsbeispiels einige Modifikationen hinsichtlich der Kopplung und Entkopplung von Antriebsglied 5 und Kolbenstange 15 auf. Das Antriebsglied 5 und die Kolbenstange 15 selbst und auch das grundsätzliche Zusammenwirken beim Koppeln und Entkoppeln sind jedoch gleich geblieben. Die der Funktion nach gleichen Komponenten sind mit den gleichen Bezugsziffern wie im ersten Ausführungsbeispiel versehen. Um auf Modifikationen hinzuweisen, sind die betreffenden Komponenten mit den gleichen, aber apostrophierten Bezugsziffern versehen.

Figur 11 zeigt das Injektionsgerät im Ruhezustand, in dem das Antriebsglied 5 von der Kolbenstange 15 entkoppelt ist. Das erste Gehäuseteil 1 ist mit einer mit dem Gehäuseteil 4 verbundenen Schutzkappe 37 abgedeckt, die für die Produktverabreichung abgenommen wird. Im Unterschied zu dem ersten Ausführungsbeispiel wird der Kupplungseingriff zwischen dem modifizierten Kupplungseingangsglied 6' und dem modifizierten Kupplungszwischenglied 7' hergestellt und gelöst.

Figur 12 zeigt das Injektionsgerät des zweiten Ausführungsbeispiels im gekoppelten Zustand, der durch die Beaufschlagung des Auslöseelements 16 und damit des Antriebsglieds 5 und des Kupplungseingangsglieds 6' durch eine in die Vortriebsrichtung V wirkende Antriebskraft hergestellt wird. Allerdings wurde wie bereits bei der korrespondierenden Figur 3 des ersten Ausführungsbeispiels noch keine Dosis oder zum Primen nur eine geringe Dosis von wenigen Einheiten ausgewählt. Die Schutzkappe 37 wurde durch ein Gehäuseteil 38 ersetzt, das auf das Gehäuseteil 4 aufgesetzt und mit diesem verschnappt ist. Das Gehäuseteil 38 lagert einen Nadelschutz 39, vorzugsweise in Form einer Nadelschutzhülse, gegen die Vortriebsrichtung V federnd beweglich. Bei einem Einstechen der nicht dargestellten Injektionsnadel federt der Nadelschutz 39 gegen die Vortriebsrichtung V in das Gehäuseteil 38 ein; in Umkehrung dieser Bewegung sticht die Nadel durch eine distale Öffnung des Nadelschutzes 39 vor.

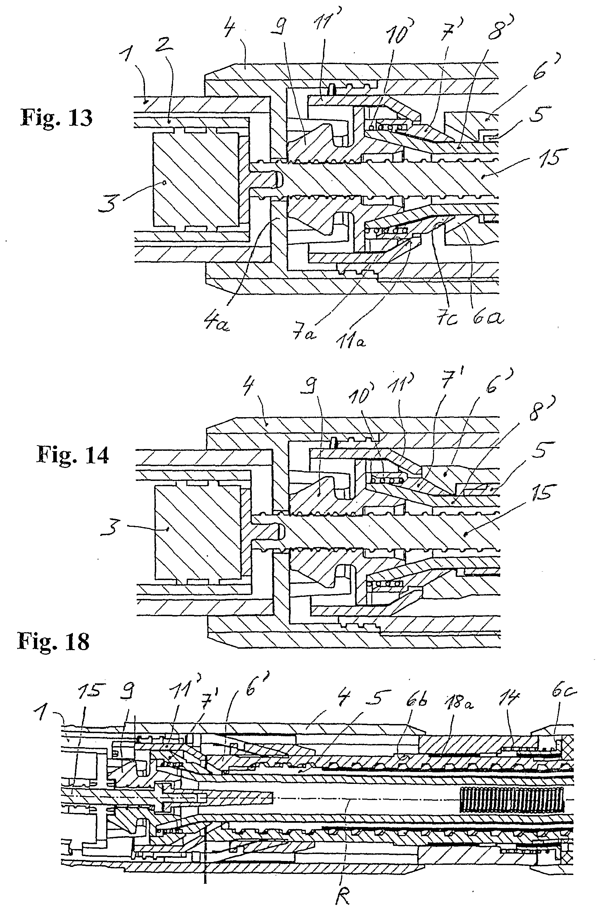

Die Figuren 13 und 14 zeigen den Bereich des Kupplungseingriffs im Detail, wobei Figur 13 für den entkoppelten Zustand und Figur 14 den gekoppelten Zustand steht. Im Unterschied zum ersten Ausführungsbeispiel weisen die Eingriffselemente 6a und 7c, zwischen denen der Kupplungseingriff hergestellt wird, zu der Vortriebsrichtung V eine Neigung auf. Im Ausführungsbeispiel sind die Eingriffselemente 6a und 7c je in der Art eines um die Gewindeachse der Kolbenstange 15 umlaufenden Kegelzahnkranzes gebildet.

Das Kupplungseingangsglied 6' bildet dabei seine Eingriffselemente 6a an seinem distalen Ende als Innenkonus, und das Kupplungszwischenglied 7' bildet die Eingriffselemente 7c an seinem proximalen Ende als Außenkonus. Die konischen Eingriffsflächen sind zueinander kongruent und liegen einander axial zugewandt mit dem lichten Abstand X unmittelbar gegenüber. Die Kupplungsflächen könnten statt konisch auch kongruent konvex/konkav geformt sein.

Das Kupplungszwischenglied 7' ist im Unterschied zum ersten Ausführungsbeispiel axial bewegbar und in jeder Axialposition verdrehgesichert mit dem Kupplungsausgangsglied 9 in einem Eingriff. Es ist wieder als Hülsenteil gebildet und axial gleitverschiebbar auf dem Kupplungsausgangsglied 9 gelagert. Hierfür durchgreift es die axial entsprechend geschlitzte Kupplungshülse 8', was in den Figuren allerdings nicht sichtbar ist. Die verdrehgesicherte Verbindung wird formschlüssig über Eingriffselemente geschaffen, die als axial gerade Verzahnungen gebildet sind. Das nach Ausbildung und Einbau gleiche, hinsichtlich seiner Funktion jedoch reduzierte Rückstellglied 10' ist wie im ersten Ausführungsbeispiel zwischen dem Kupplungsausgangsglied 9 und dem Kupplungszwischenglied 7' gespannt und beaufschlagt letzteres gegen die Vortriebsrichtung V mit einer Elastizitätskraft. Im entkoppelten Zustand, in dem das Kupplungseingangsglied 6', wie in Figur 13 gezeigt, gegen die Vortriebsrichtung V von dem Kupplungszwischenglied 7' abgerückt ist, drückt das Rückstellglied 10' das Kupplungszwischenglied 7' in den verdrehgesicherten Eingriff mit dem Entkopplungsglied 11'. Die entsprechenden Eingriffselemente sind wieder mit 7a und 11a bezeichnet. Auch die Eingriffselemente 7a und 11a sind als kegelige Zahnkränze gebildet. Der Eingriff von Kupplungszwischenglied 7' und Entkopplungsglied 11' kann alternativ rein reibschlüssig sein. Die Eingriffselemente 7a und 11a weisen in diesem Fall einander zugewandte, kongruente Reibflächen auf, im Ausführungsbeispiel wären dies die einander zugewandten Konusflächen.

Eine weitere Modifikation besteht bei dem Dosierglied 18'. Im Unterschied zu dem Dosierglied 18 des ersten Ausführungsbeispiels ist das Dosierglied 18' relativ zu dem Gehäuseteil 4 in Richtung der Kupplungsbewegung X, im Ausführungsbeispiel die axiale Richtung, nicht bewegbar. Stattdessen ist das Kupplungseingangsglied 6' zwar wieder verdrehgesichert, aber axial beweglich mit dem Dosierglied 18' verbunden. Der verdrehgesicherte Eingriff zwischen dem Kupplungseingangsglied 6' und dem Dosierglied 18' besteht im entkoppelten Zustand von Antriebsglied 5 und Kolbenstange 15 und wird im Verlaufe des Kupplungshubs X gelöst, nämlich unmittelbar bevor die verdrehgesicherte Verbindung zwischen dem Kupplungsausgangsglied 9 und dem Gehäuseteil 4 gelöst wird. Für diesen Eingriff sind das Kupplungseingangsglied 6' und das Dosierglied 18' mit Eingriffselementen 6b und 18a versehen, die an radial einander zugewandten Mantelflächen der beiden Glieder 6' und 18' in der Art von Nuten und Federn gebildet sind. Bezüglich der verdrehgesicherten Verbindung zwischen dem Kupplungseingangsglied 6' und dem Dosierglied 18' sei auch auf die Figuren 11 und 12 verwiesen. In dem in Figur 11 dargestellten entkoppelten Zustand besteht die verdrehgesicherte Verbindung, und in dem in Figur 12 dargestellten gekoppelten Zustand ist sie gelöst.

Noch ein Unterschied zum ersten Ausführungsbeispiel besteht hinsichtlich der Halteeinrichtung. Das Rückstellglied 10' hat im zweiten Ausführungsbeispiel keine, die Kupplungsglieder 6' und 9 voneinander trennende Wirkung. Die Halteeinrichtung des zweiten Ausführungsbeispiels umfasst ein Rückstellglied 14, ein Stützstruktur 6c und das Dosierglied 18'. Das Rückstellglied 14 beaufschlagt das Kupplungseingangsglied 6' über das Stützstruktur 6c mit einer elastischen Rückstellkraft, die der Kupplungsbewegung X des Kupplungseingangsglieds 6' entgegenwirkt. In Richtung der Kupplungsbewegung X, die in den Ausführungsbeispielen mit der Vortriebsrichtung V zusammenfällt, ist das Rückstellglied 14 an dem Dosierglied 18' abgestützt, das hierfür eine Abstützschulter bildet. Das Stützstruktur 6c ist in und gegen die Richtung der Kupplungsbewegung X unbeweglich mit dem Kupplungseingangsglied 6' verbunden. Es ist als kurze Hülse mit einem Außenflansch gebildet, an dem sich das Rückstellglied 14 abstützt. Gegen die Richtung der Kupplungsbewegung X liegt das Stützstruktur 6c in Bezug auf das Gehäuseteil 4 auf Anschlag. Durch die Kupplungsbewegung X wird das Kupplungseingangsglied 6' gegen die elastische Rückstellkraft des Rückstellglieds 14 in den Kupplungseingriff mit dem Kupplungszwischenglied 7' bewegt. Das Rückstellglied 14 ist wie im ersten Ausführungsbeispiel als eine in Richtung der Kupplungsbewegung X mit einer Druckkraft beaufschlagte Druckfeder gebildet.

Die Arbeitsweise der modifizierten Kupplung 6'-11', 14' ist die gleiche wie die Kupplung des ersten Ausführungsbeispiels. So ist das Kupplungsausgangsglied 9 in dem entkoppelten Zustand über die Kupplungshülse 8', das Kupplungszwischenglied 7' und das Entkopplungsglied 11' verdrehgesichert mit dem Gehäuseteil 4 verbunden. Durch Betätigung des Injektionsknopfs 16 und damit einhergehend der Ausführung des Kupplungshubs X (Figur 11) wird der Kupplungseingriff hergestellt, im zweiten Ausführungsbeispiel zwischen dem Kupplungseingangsglied 6' und dem Kupplungszwischenglied 7'. In der ersten Phase des Kupplungshubs X greifen die Eingriffselemente 6a und 7c ineinander, so dass das Kupplungseingangsglied 6' über das Kupplungszwischenglied 7' und die Kupplungshülse 8' verdrehgesichert mit dem Kupplungsausgangsglied 9 verbunden ist. Erst nach Herstellung des verdrehgesicherten Eingriffs wird durch das in die Vortriebsrichtung V drückende Kupplungseingangsglied 6' das Kupplungsglied 7' aus dem Eingriff mit dem Entkopplungsglied 11' bewegt, so dass das Kupplungsausgangsglied 9 frei um die mit der Kolbenstange 15 gebildete Gewindeachse R drehen kann und der Kupplungseingriff vollständig hergestellt ist.

Figur 14 zeigt das Injektionsgerät im gekoppelten Zustand, d.h. im Kupplungseingriff.

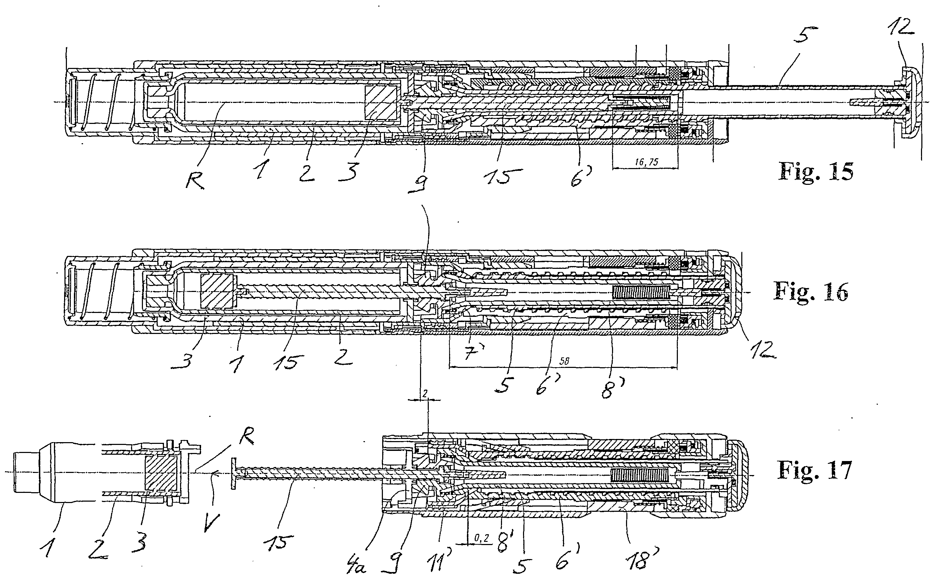

Die Figuren 15 und 16 entsprechen den Figuren 6 und 7 des ersten Ausführungsbeispiels, so dass darauf verwiesen werden kann.

Figur 17 zeigt das Injektionsgerät des zweiten Ausführungsbeispiels während des Austauschs des Reservoirs 2. Nachdem das Reservoir 2, wie in Figur 16 gezeigt, entleert ist, wird das Gehäuseteil 1 von dem Gehäuseteil 4 gelöst, wodurch das Entkopplungsglied 11' in die Entkopplungsposition bewegt wird. Diese Funktion entspricht vollkommen derjenigen des Entkopplungsglieds 11 des ersten Ausführungsbeispiels, so dass auf die dortigen Erläuterungen und die Figuren 8-10 verwiesen werden kann.

In dem in Figur 17 gezeigten Zustand nimmt das Gehäuseteil 1 bereits das neue Reservoir 2 auf. Um das Gehäuseteil 1 mit dem Gehäuseteil 4 zu verbinden, kann das Gehäuseteil 1 mit dem das Reservoir 2 proximal verschließenden Kolben 3 auf das Gehäuseteil 4 zu bewegt werden. Die frei aus dem Gehäuseteil 4 vorragende Kolbenstange 15 wird durch den andrückenden Kolben 3 im Gewindeeingriff mit dem frei drehbaren, aber axial festgelegten Kupplungsausgangsglied 9 zurückbewegt. Wegen der verdrehgesicherten Linearführung 4a, die im zweiten Ausführungsbeispiel von einer verdrehgesichert in das Gehäuseteil 4 eingesetzten Kupplungsaufnahme gebildet wird, vollführt die Kolbenstange 15 bei dem Zurückschieben eine axiale Linearbewegung, während das Kupplungsausgangsglied 9 gemeinsam mit der Kupplungshülse 8' um die gemeinsame Gewindeachse frei dreht. Anstatt die Kolbenstange 15 gegen den Kolben 3 drückend zurück zu bewegen, kann die Kolbenstange 15 auch vorher durch Druck unmittelbar auf ihren Stempel zurückbewegt werden.

Figur 18 zeigt den Kupplungsbereich mit dem in der Entkopplungsposition befindlichen Entkopplungsglied 11' im Detail. Die Funktion des Entkopplungsglieds 11' entspricht derjenigen des ersten Ausführungsbeispiels, nämlich Blockierung des Kupplungseingangsglieds 6' in der abgerückten Axialposition.

Im zweiten Ausführungsbeispiel werden die Dosierbewegung und die Antriebsbewegung ebenfalls über das Kupplungseingangsglied 6' und ein Anzeigekopplungsglied 22 in ein Getriebe der Dosisanzeige 20' eingeleitet. Auch das Anzeigekopplungsglied 22 ist verdrehgesichert mit dem Kupplungseingangsglied 6' verbunden und relativ zu dem Gehäuseteil 4 in und gegen die Richtung der Kupplungsbewegung X nicht beweglich.

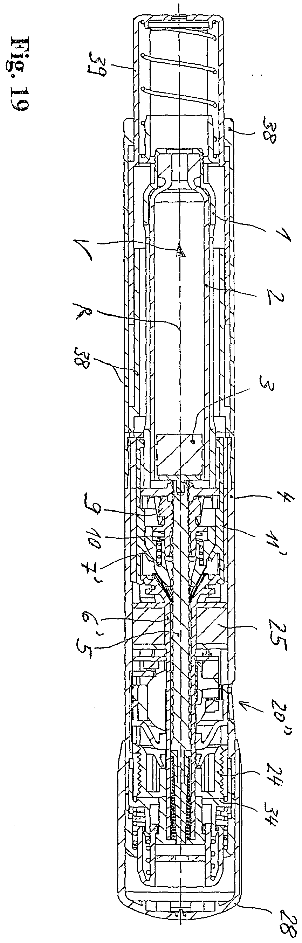

Die Figuren 19 bis 24 zeigen als drittes Ausführungsbeispiel ein Injektionsgerät, bei dem bei der Verabreichung die Antriebskraft für die Produktausschüttung nicht manuell, sondern von einem Antriebsglied 25 aufgebracht wird, das als Antriebsfeder gebildet ist. Das Antriebsglied 25 wird durch Einstellung der zu verabreichenden Dosis gespannt. Die bei der Dosiseinstellung aufgenommene Federenergie wird bei Auslösung des Geräts freigesetzt und in den Vortrieb der Kolbenstange 15 umgewandelt.

Figur 19 zeigt das Injektionsgerät des dritten Ausführungsbeispiels komplett mit dem montierten Gehäuseteil 38 und dem darin entgegen der Vortriebsrichtung V gegen die Kraft einer Rückstellfeder gleitverschiebbar aufgenommenen Nadelschutz 39.

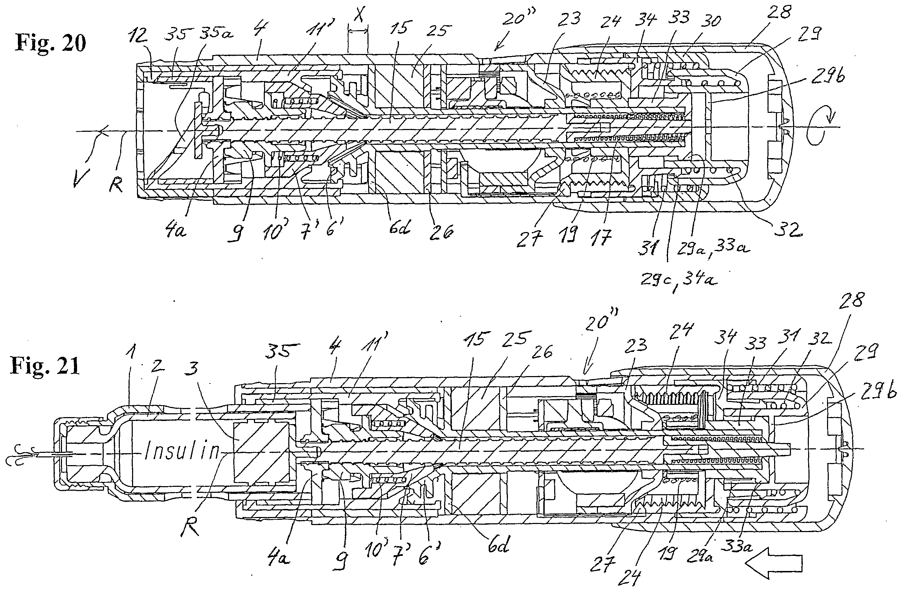

Die Figuren 20 und 21 zeigen das Gehäuseteil 4 mit den darin aufgenommenen Komponenten des Injektionsgeräts, Figur 20 in einem den vorhergehenden Ausführungsbeispielen vergleichbaren Ruhezustand, in dem die Dosis eingestellt werden kann, und Figur 21 im Kupplungseingriff. Soweit nachfolgend nichts anderes gesagt wird, werden insbesondere die Figuren 20 und 21 in Bezug genommen.

Das Antriebsglied 25 ist eine als Drehfeder wirkende Spiralfeder mit Federwindungen, die um die Gewindeachse R des Gewindeeingriffs zwischen dem Kupplungsausgangsglied 9 und der Kolbenstange 15 umlaufen. Die Federwindungen sind radial zu der Gewindeachse R übereinander angeordnet; sie weisen zu der Gewindeachse die Steigung Null auf. Ein inneres Ende der Federwindungen ist an dem Kupplungseingangsglied 6' befestigt, und ein äußeres Ende ist an einer Stützstruktur 26 befestigt, die mit dem Gehäuseteil 4 in Richtung der Kupplungsbewegung X bewegbar, aber verdrehgesichert verbunden ist. Die Stützstruktur 26 ist andererseits in und gegen die Richtung der Kupplungsbewegung X nicht bewegbar mit dem Kupplungseingangsglied 6' verbunden. Das Kupplungseingangsglied 6' ist relativ zu der Stützstruktur 26 um die Gewindeachse R drehbar. Eine weitere Stützstruktur 6d ist mit dem Kopplungseingangsglied 6' in und gegen die Richtung der Kupplungsbewegung X nicht bewegbar verbunden; im Ausführungsbeispiel sind das Kupplungseingangsglied 6' und die Stützstruktur 6d in einem Stück gebildet. Das Antriebsglied 25 wird von den Stützstrukturen 6d und 26 axial eingefasst.

Die Kupplung entspricht in ihrer Funktionsweise derjenigen des zweiten Ausführungsbeispiels, so dass für die Kupplungsglieder 6'-10' und das Entkopplungsglied 11' die gleichen Bezugszeichen verwendet werden. Im Unterschied zu der Kupplung des zweiten Ausführungsbeispiels ist allerdings die dortige Kupplungshülse 8' entfallen. Das Kupplungszwischenglied 7' ist unmittelbar mit dem Kupplungsausgangsglied 9 in einem die rotatorische Antriebsbewegung des Kupplungseingangsglieds 6' auf das Kupplungsausgangsglied 9 übertragenden Eingriff.

Mit 20" ist eine Dosisanzeige bezeichnet, die über ein Anzeigekopplungsglied 23 mit dem Kupplungseingangsglied 6' gekoppelt ist, und wie bereits die Anzeigekopplungsglieder 21 und 22 der anderen Ausführungsbeispiele verdrehgesichert mit dem Kupplungseingangsglied 6' verbunden ist. In und gegen die Richtung der Kupplungsbewegung X ist das Anzeigekopplungsglied 23 relativ zu dem Gehäuseteil 4 nicht bewegbar. Wie bereits im ersten und zweiten Ausführungsbeispiel besteht die verdrehgesicherte Verbindung des Anzeigekopplungsglieds 23 sowohl im ausgekuppelten als auch im eingekuppelten Zustand der Vorrichtung.

Um das Kupplungseingangsglied 6' für die Einstellung der Dosis und während der Aufbewahrung an der rotatorischen Antriebsbewegung zu hindern und das Antriebsglied 25 im gespannten Zustand zu halten, ist zwischen dem Kupplungsgehäuse 6' und dem Gehäuseteil 4 eine Verdrehsperre gebildet. Die Verdrehsperre besteht in der dargestellten Halteposition der Kupplungsglieder 6', 7' und 9 zwischen einem ersten Sperrglied 24 und einem zweiten Sperrglied 34. Das Sperrglied 24 ist verdrehgesichert mit dem Kupplungseingangsglied 6' verbunden. Das Sperrglied 34 ist verdrehgesichert mit dem Gehäuseteil 4 verbunden, aber relativ zu dem Gehäuseteil 4 und dem Kupplungseingangsglied 6' in und gegen die Richtung der Kupplungsbewegung X bewegbar. Die Sperrglieder 24 und 34 bilden mit ihren im Sperreingriff einander kontaktierenden Stirnflächen eine Ratsche, die eine das Antriebsglied 25 spannende Rotationsbewegung des Kupplungseingangsglieds 6' zuläßt und eine Rotationsbewegung in die Gegenrichtung verhindert.

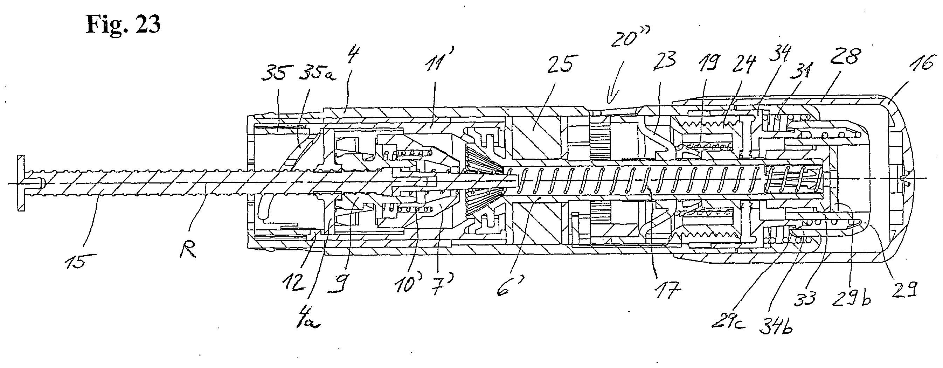

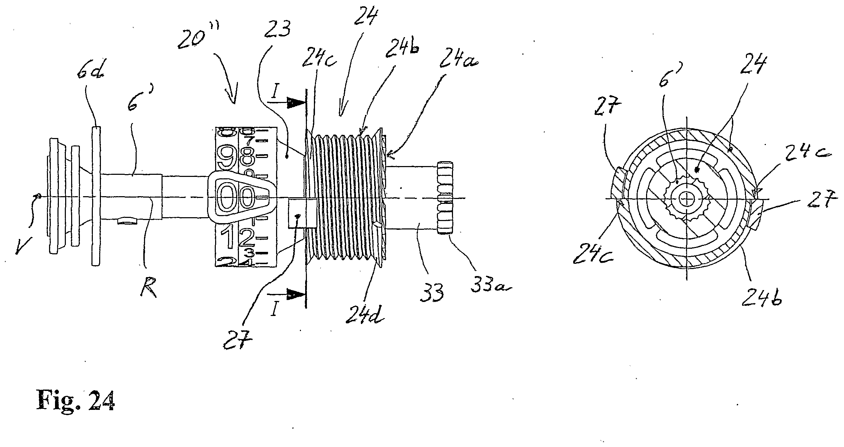

Figur 24 zeigt das Kupplungseingangsglied 6' mit dem darauf verdrehgesichert gelagerten Sperrglied 24, dem verdrehgesichert mit dem Kupplungseingangsglied 6' verbundenen Anzeigekopplungsglied 23 und einem mit dem Eingangsglied 6' nicht beweglich verbundenen Verbindungsteil 33. Das Anzeigekopplungsglied 23 bildet einen Einer-Zählring der Dosisanzeige 20" und ist mit einem Zehner-Zählring zur Anzeige der eingestellten Dosis in geeigneter Weise gekoppelt. Das Sperrglied 24 ist an einer dem Sperrglied 34 zugewandten, proximalen Stirnseite mit um die Achse R gleichmäßig angeordneten Sperrzähnen 24a versehen, die mit Gegenzähnen des Sperrglieds 34 im Sperreingriff zusammenwirken, um die Verdrehsperre bezüglich der Antriebsbewegung zu bilden. Das Sperrglied 24 ist für eine Zweitfunktion im Zusammenhang mit der Dosierung und Ausschüttung an einer Mantelaußenfläche mit einem Gewinde 24b versehen, dessen Gewindeachse mit der Gewindeachse R der Kolbenstange 15 zusammenfällt. In das Gewinde 24b greift ein Stoppglied 27 ein. Das Stoppglied 27 ist parallel zu der Gewindeachse R linear bewegbar geführt, im Ausfiihrungsbeispiel in einer axialen Nut an der Innenmantelfläche des Gehäuseteils 4. Das Sperrglied 24 bildet für das Stoppglied 27 einen Verdrehanschlag 24c, der die den Vortrieb der Kolbenstange 15 bewirkende Antriebsbewegung des Kupplungseingangsglieds 6' begrenzt. Es bildet für das Stoppglied 27 einen weiteren Verdrehanschlag 24d, der die ausschüttbare und einstellbare Maximaldosis bestimmt. Auf der anderen Seite der Gewindeachse R ist dem in der Ansicht der Figur 23 erkennbaren Stoppglied 27 gegenüberliegend ein weiteres Stoppglied 27 angeordnet, das in gleicher Weise mit zwei weiteren Verdrehanschlägen 24c und 24d zusammenwirkt. Das Gewinde 24d ist doppelgängig. Die Stoppglieder 27 gelangen gleichzeitig auf Anschlag gegen die jeweils zugeordneten Verdrehanschläge 24c und 24d, wie in der Querschnittsdarstellung der Figur 23 für die Verdrehanschläge 24c erkennbar ist. Die Verdrehanschläge 24c bestimmen eine Nulldosisposition und die Verdrehanschläge 24d eine Maximaldosisposition.

Die Halteeinrichtung ist im dritten Ausführungsbeispiel in einer dritten Variante gebildet. Sie umfasst ein Rückstellglied 19, ferner das Anzeigekopplungsglied 23 und das Sperrglied 24. Das Rückstellglied 19 ist in Richtung der Kupplungsbewegung X über das Anzeigekopplungsglied 23 an dem Gehäuseteil 4 und gegen die Richtung der Kupplungsbewegung X an dem Sperrglied 24 abgestützt. Das Rückstellglied 19 drückt das Sperrglied 24 auf Anschlag gegen das Verbindungsteil 33. Da das Verbindungsteil 33 in und gegen die Richtung der Kupplungsbewegung X nicht beweglich mit dem Kupplungseingangsglied 6' verbunden ist, übt das Rückstellglied 19 somit über das Sperrglied 24 und das Verbindungsteil 33 auf das Kupplungseingangsglied 6' eine gegen die Richtung der Kupplungsbewegung X wirkende elastische Rückstellkraft aus, die das Kupplungseingangsglied 6' in der aus dem Kupplungseingriff ausgerückten Halteposition hält. Es wirkt wieder als Druckfeder. Das Sperrglied 24 ist ein Hülsenteil mit einem das Gewinde 24b bildenden äußeren Mantel, einem der verdrehgesicherten Lagerung auf dem Kupplungseingangsglied 6' dienenden inneren Mantel und einem die beiden Mäntel verbindenden Boden, an dem die Sperrzähne 24a gebildet sind. In das derart topfförmige Sperrglied 24 ragt das Rückstellglied 19 und stützt sich an dem Boden des Sperrglieds 24 ab.

Das Rückstellglied 19 drückt das Sperrglied 24 nicht nur auf Anschlag gegen das Verbindungsteil 33, sondern auch auf einen Anschlag gegen das Gehäuseteil 4. Dieser weitere Anschlag verhindert, dass das Sperrglied 24 über die in Figur 20 eingenommene Halteposition hinaus gegen die Richtung der Kupplungsbewegung X bewegt werden kann. Das Sperrglied 24 ist somit relativ zu dem Kupplungseingangsglied 6' gegen die rückstellende Elastizitätskraft des Rückstellglieds 19 in Richtung der Kupplungsbewegung X bewegbar. Umgekehrt ist das Kupplungseingangsglied 6' relativ zu dem auf Anschlag gegen das Gehäuseteil 4 liegenden Sperrglied 24 gegen die Richtung der Kupplungsbewegung X bewegbar.

Die zwischen der Kolbenstange 15 und dem Verbindungsteil 33 gespannte Ausgleichsfeder 17 unterstützt das Rückstellglied 19 bei dessen Funktion, das Kupplungseingangsglied 6' in der Halteposition zu halten. Die Ausgleichsfeder 17 könnte für das Ausrücken der Kupplungsglieder 6', 7' und 9 im Grunde das Rückstellglied 19 ersetzen. Bevorzugt ist sie jedoch so schwach, dass sie zumindest nach einer teilweisen Entspannung die Kupplungsglieder 6'-9 nicht mehr mit ausreichender Sicherheit in der Halteposition und die Kupplung somit im ausgekuppelten Zustand halten kann.

Für die Auslösung des Antriebsglieds 25 ist ein Auslöseelement 28 vorgesehen. Das Auslöseelement 28 ist relativ zu dem Gehäuseteil 4 in Richtung der Kupplungsbewegung X, im Ausführungsbeispiel die Vortriebsrichtung V bzw. die distale Richtung, translatorisch und um die Rotationsachse R des Kupplungseingangsglieds 6', die im Ausführungsbeispiel mit der Gewindeachse R der Kolbenstange 15 zusammenfällt, rotatorisch bewegbar und wird bei diesen beiden Bewegungen von dem Gehäuseteil 4 geführt. Durch die translatorische Bewegung in die distale Richtung werden der Kupplungseingriff zwischen dem Kupplungseingangsglied 6' und dem Kupplungszwischenglied 7' hergestellt und die Verdrehsperre zwischen den Sperrgliedern 24 und 34 gelöst und das Antriebsglied 25, d.h. die Ausschüttung, hierdurch ausgelöst. Die translatorische Bewegung in die Vortriebsrichtung V wird im Folgenden daher auch als Auslösebewegung bezeichnet.

Das Auslöseelement 28 bildet in einer weiteren Funktion das Dosierglied des dritten Ausführungsbeispiels. Durch die rotatorische Bewegung des Auslöseelements 28 relativ zu dem Gehäuseteil 4 wird über mehrere Zwischenglieder die mit dem nächsten Ausschüttvorgang ausschüttbare Produktdosis eingestellt. Diese Bewegung wird im Folgenden auch als Dosierbewegung bezeichnet. Aus der Nulldosisposition, die in Figur 20 dargestellt ist und durch Anschlag der Stoppglieder 27 an den die Antriebsbewegung des Kupplungseingangsglieds 6' begrenzenden Verdrehanschlägen 29c des Sperrglieds 24 bestimmt wird, kann die Dosis durch Verdrehen des Auslöseelements 28 in Richtung des eingezeichneten Drehrichtungspfeils, die Dosierrichtung, eingestellt werden. Die rotatorische Dosierbewegung des Auslöseelements 28 wird über ein Innenteil 29, das verdreh- und verschiebegesichert mit dem Auslöseelement 28 verbunden oder einstückig damit gebildet ist, und das Verbindungsteil 33 auf das Kupplungseingangsglied 6' übertragen. Für die Übertragung sind das Innenteil 29 und das Verbindungsteil 33 miteinander in einem verdrehgesicherten Eingriff, und das Verbindungsteil 33 ist mit dem Kupplungseingangsglied 6' verdrehgesichert verbunden. Für die Verdrehsicherung sind das Innenteil 29 und das Verbindungsteil 33 mit einer Innenverzahnung 29a und einer Außenverzahnung 33a versehen, die im Ruhezustand des Geräts ineinander greifen und axial gegeneinander verschiebbar sind.

Das Auslöseelement 28 ist bedienerfreundlich im proximalen Endbereich des Gehäuseteils 4 angeordnet. Sein äußeres Hülsenteil umgibt das Gehäuseteil 4. Ein Boden des Auslöseelements 28 bildet ein proximales Ende des Injektionsgeräts. Für die Einstellung der Dosis ist das Auslöseelement 28 wie ein Drehknopf bedienbar und weist hierfür an seiner äußeren Mantelfläche eine Riffelung auf. Für die Auslösung ist es wie ein Druckknopf bedienbar. Bei der Dosierbewegung verrastet das Auslöseelement 28 den Dosiseinheiten entsprechend in diskreten Drehwinkelpositionen mit dem Gehäuseteil 4.

Von dem Innenteil 29 ragt ein Anschlagelement 29b einer proximalen Stirnfläche des Verbindungsteils 33 zugewandt nach radial einwärts ab. Im Ruhezustand des Geräts verbleibt zwischen dem Verbindungsteil 33 und dem Anschlagelement 29b ein lichter Abstand, der gerade so groß ist, dass bei der Auslösebewegung des Auslöseelements 28 die Verdrehsicherung zwischen dem Innenteil 29 und dem Verbindungsteil 33 sich löst, bevor das Anschlagelement 29b die Relativbewegung des Auslöselements 28 relativ zu dem Verbindungsteil 33 durch Anschlagkontakt beendet.

Das zweite Sperrglied 34 wird mittels einer Sperrfeder 31 in den Sperreingriff mit dem Sperrglied 24 gespannt. Die Sperrfeder 31 stützt sich hierfür in Richtung der Kupplungsbewegung X an dem Sperrglied 34 und der Kupplungsbewegung X entgegen an einem Gehäuseteil 30 ab, das mit dem Gehäuseteil 4 fest verbunden ist. Eine zwischen dem Innenteil 29 und dem Sperrglied 34 angeordnete weitere Feder 32 spannt das Auslöseelement 28 relativ zu dem Sperrglied 34 in eine proximale Endposition. Das Sperrglied 34 wird von dem Gehäuseteil 4 verdrehgesichert axial geführt. Das Gehäuseteil 4 bildet für die Bewegbarkeit des Sperrglieds 34 einen distalen und einen proximalen Anschlag.

In dem in Figur 20 dargestellten Ruhezustand stellt der Benutzer die Dosis ein, indem er das Auslöselement 28 in die Dosierrichtung verdreht. Das Auslöseelement 28 nimmt bei dieser rotatorischen Dosierbewegung über die Verdrehsicherung 29a, 33a das Verbindungsteil 33 mit, das seinerseits das Kupplungseingangsglied 6' mitnimmt, das somit die gleiche rotatorische Dosierbewegung wie das Auslöseelement 28 vollführt. Durch die Rotation des Kupplungseingangsglieds 6' wird das Antriebsglied 25 gespannt. Das Stoppglied 27 wandert im Eingriff mit dem Gewinde 24b des Sperrglieds 24 von dem die Nulldosis bestimmenden Anschlag 24c des Gewindes 24b in Richtung des die Maximaldosis bestimmenden Anschlags 24d (Fig. 24).

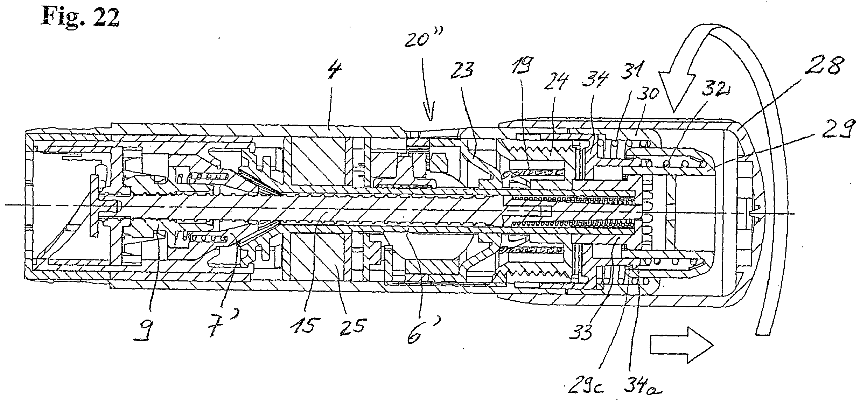

Das Injektionsgerät bietet auch eine bequeme Möglichkeit der Dosiskorrektur, wie anhand des Vergleichs der Figuren 20 und 22 deutlich wird. Sollte der Benutzer versehentlich eine zu hohe Dosis eingestellt haben, kann er die Dosis durch Zurückdrehen des Kupplungseingangsglieds 6' korrigieren. Für die Dosiskorrektur zieht er das Auslöseelement 28 in die proximale Richtung. Diese Ausrückbewegung des Auslöseelements 28 ist in Figur 22 durch einen Pfeil angedeutet, ebenso wie die Drehrichtung für die Korrektur. Das Innenteil 29 und das Sperrglied 34 sind in dem Ruhezustand des Geräts in Bezug auf eine Bewegung in die proximale Richtung in einem Mitnahmeeingriff. Die entsprechenden Mitnehmer sind mit 29c und 34a bezeichnet. Die von dem Innenteil 29 gebildeten Mitnehmer 29c und die von dem Sperrglied 34 gebildeten Mitnehmer 34a hintergreifen einander und bilden für die Ausrückbewegung des Auslöseelements 28 eine Verhakung. Durch Zug an dem Auslöseelement 28 wird somit das Sperrglied 34 gegen die Kraft der Sperrfeder 31 ebenfalls in die proximale Richtung bewegt und löst sich dabei aus dem Sperreingriff mit dem Sperrglied 24, das gegen das Gehäuseteil 4 auf Anschlag ist. Sobald die Verdrehsperre gelöst ist, kann der Benutzer mittels einer Rückdrehbewegung des Auslöseelements 28 und den nach wie vor bestehenden verdrehgesicherten Eingriff des Innenteils 29 mit dem Verbindungsteil 33 die Dosis korrigieren. Sobald der Benutzer das Auslöseelement 28 freigibt, schnappt es zusammen mit dem Sperrglied 34 unter der Wirkung der Sperrfeder 31 in die distale Richtung zurück und das Sperrglied 34 dadurch wieder in den Sperreingriff mit dem Sperrglied 24. Bei der Rückdrehbewegung hält der Benutzer zweckmäßigerweise weiterhin das Auslöseelement 28 fest, was durch die Drehwinkelrastpositionen des Auslöseelements 28 erleichtert wird. Grundsätzlich kann er es jedoch auch zurückschnappen lassen und gegebenenfalls wieder aufdosieren.

Nach Einstellung der gewünschten Dosis wird das Gerät an der gewünschten Stelle der Verabreichung auf die Haut aufgesetzt, und die Injektionsnadel wird eingestochen. Das Auslöseelement 28 übernimmt für das Einstechen der Nadel eine weitere Funktion, wofür es mit dem Nadelschutz 39 (Figur 19) gekoppelt ist.

In einer ersten Phase des Einstechens drückt der Benutzer das Injektionsgerät gegen die Haut, so dass sich der Nadelschutz 39 relativ zu dem Gehäuseteil 38 in die distale Richtung bewegt. Durch diesen ersten Teil der Bewegung des Nadelschutzes 39 wird die Injektionsnadel allerdings noch nicht freigelegt, ihre Spitze steht vielmehr weiterhin hinter dem Nadelschutz 39 zurück. Der Nadelschutz 39 gelangt in dieser ersten Phase des Einstechvorgangs auf Anschlag gegen ein Widerstandselement, so dass er sich relativ zu dem Gehäuseteil 38 nicht weiter in die distale Richtung bewegen kann. Bei weiterhin auf das Injektionsgerät in Richtung Haut ausgeübtem Druck drückt der Benutzer das Auslöseelement 28 in die proximale Richtung. Im Verlaufe dieser ersten Phase seiner Auslösebewegung löst das Auslöseelement 28 einen Anschlagkontakt zwischen dem Nadelschutz 39 und dem Widerstandselement, so dass das Injektionsgerät und damit zusammen die Injektionsnadel relativ zu dem Nadelschutz 39 in Richtung auf die Haut bewegt werden und die Injektionsnadel einsticht. Bezüglich der Funktion des Auslöseelements 28 für das Einstechen der Nadel wird die von der Anmelderin unter dem gleichen Anmeldedatum eingereichte Patentanmeldung "Aufsatzmodul für eine Injektionsvorrichtung mit einer Eingriffssteuerung für ein Nadelabdeckelement" in Bezug genommen.

Sobald die Injektionsnadel subkutan platziert ist, kann durch weiteren Druck auf das Auslöseelement 28 das Antriebsglied 25 ausgelöst und die Ausschüttung bewirkt werden. In der zweiten Phase der Auslösebewegung des Auslöseelements 28, die sich an die Einstechphase anschließt, wird das Auslöseelement 28 und damit das Innenteil 29 gegen den Druck der Feder 32 relativ zu dem Verbindungsteil 33 weiter in die distale Richtung gedrückt, so dass sich die Verdrehsicherung 29a, 33a löst. Das Auslöseelement 28 kann leer drehen. Sobald die Verdrehsicherung 29a, 33a gelöst ist, gelangt das Anschlagelement 29b in Anschlagkontakt mit dem Verbindungsteil 33. In der sich nun anschließenden dritten Phase der Auslösebewegung drückt das Auslöseelement 28 über das Anschlagelement 29b das Verbindungsteil 33 und damit das Kupplungseingangsglied 6' in die Richtung der Kupplungsbewegung X, im Ausführungsbeispiel in die Vortriebsrichtung V. Unter der Einwirkung der Federkraft der Sperrfeder 31 folgt das Sperrglied 34 dieser Bewegung, bis es auf Anschlag gegen das Gehäuseteil 4 gelangt. Bevor das Sperrglied 34 die Anschlagposition erreicht, gelangt das Kupplungseingangsglied 6' in den Kupplungseingriff mit dem Kupplungszwischenglied 7'. Das Kupplungseingangsglied 6' drückt das Kupplungszwischenglied 7' gegen die Kraft des Rückstellglieds 10' aus dem reibschlüssigen Sperreingriff mit dem Entkopplungsglied 11'. Nachdem der Sperreingriff zwischen den Konusflächen der beiden Glieder 7' und 11' gelöst und damit der Kupplungseingriff vollständig hergestellt ist, gelangt das Sperrglied 34 auf Anschlag mit dem Gehäuseteil 4. In der sich nun anschließenden letzten Phase der Auslösebewegung drückt das Auslöseelement 28 über das Verbindungsteil 33 das Sperrglied 24 aus dem Sperreingriff mit dem Sperrglied 34.

Sobald die von den Sperrgliedern 24 und 34 gebildete Verdrehsperre gelöst ist, setzt aufgrund der Antriebskraft des Antriebsglieds 25 die rotatorische Antriebsbewegung des Kupplungseingangsglieds 6' ein und wird über den Kupplungseingriff auf das Kupplungsausgangsglied 9 übertragen. Wegen ihrer verdrehgesicherten Führung in der Linearführung 4a bewegt sich die Kolbenstange 15 im Gewindeeingriff mit dem Kupplungsausgangsglied 9 in die Vortriebsrichtung V, und es wird Produkt ausgeschüttet. Diese Ausschüttbewegung wird durch Anschlag des Stoppglieds 27 an dem die Nulldosis bestimmenden Anschlag 24c des Sperrglieds 24 beendet.

Figur 21 zeigt das Injektionsgerät für den Fall der Einstellung einer Nulldosis oder einer geringen Primingdosis im eingekuppelten Zustand nach dem Lösen der Verdrehsperre 24, 34, d.h. nachdem das Auslöseelement 28 die Auslösebewegung vollständig ausgeführt hat. Die vorstehend beschriebene Auslösesequenz läuft bei vorteilhafterweise kontinuierlich auf das Auslöseelement 28 ausgeübtem Druck automatisch ab, vom Einstechen bis zur vollständigen Ausschüttung der eingestellten Dosis.

Figur 23 zeigt das Injektionsgerät nach Entleerung des Behältnisses 2. Das Gehäuseteil 1 ist bereits von dem Gehäuseteil 4 abgenommen worden. Die Kolbenstange 15 nimmt ihre distalste Position ein. Das Entkopplungsglied 11' blockiert das Kupplungseingangsglied 6' in der von dem Kupplungszwischenglied 7' abgerückten Position. Die Funktionsweise des Entkopplungsglieds 11' entspricht derjenigen in den anderen Ausführungsbeispielen. Im Unterschied zu den beiden ersten Ausführungsbeispielen sind das Gehäuseteil 1 und das Entkopplungsglied 11' jedoch nicht unmittelbar miteinander in einem Führungseingriff, sondern über eine Adapterstruktur 36. Die Adapterstruktur 36 ist eine Hülse die in dem Gehäuseteil 4 in denen Verbindungsabschnitt in und gegen die Richtung der Kupplungsbewegung X fixiert ist, sich aber um die zentrale Längsachse R des Gehäuseteils 4 drehen kann. Die Adapterstruktur 35 bildet eine Führungskurve 36a entweder als Ausnehmung an oder Durchbrechung in ihrer dem Entkopplungsglied 11' zugewandten Mantelfläche. Die Führungskurve 35a weist den Verlauf eines Gewindeabschnitts auf. Die über den Umfang gemessene Länge und die in Bezug auf die zentrale Längsachse des Gehäuseteils 4 gemessene Steigung der Führungskurve 35a sind so bemessen, dass bei einer viertel bis halben Umdrehung der Adapterstruktur 35 relativ zu dem Entkopplungsglied 11' dieses in die in Figur 21 dargestellte Entkopplungsposition bewegt wird. Für die Erzeugung der Axialbewegung greift das Entkopplungsglied 11' mit seinem Eingriffselement 12 in die Führungskurve 35a. Insoweit wird auf die Ausführungen zum ersten Ausführungsbeispiel verwiesen.

Die Adapterstruktur 35 bildet bei dem Verbinden der Gehäuseteile 1 und 4 eine Linearführung für das Gehäuseteil 1. Das Gehäuseteil 1 wird in die Adapterstruktur 35 eingeschoben, wobei vorzugsweise ein leichter Reibschluss und dementsprechend eine Gleitführung für das Gehäuseteil 1 besteht. Das Gehäuseteil 1 ist relativ zu der Adapterstruktur 35 um die zentrale Längsachse des Gehäuseteils 4 nicht verdrehbar. Der entsprechend verdrehgesicherte Eingriff wird gleich zu Beginn des Einschiebens des Gehäuseteils 1 in die Adapterstruktur 35 hergestellt. Nachdem das Gehäuseteil 1 bis auf Anschlag gegen das Gehäuseteil 4, nämlich dessen Kupplungsaufnahme bei 4a, eingeschoben worden ist, wird das Gehäuseteil 1 relativ zu dem Gehäuseteil 4 verdreht und nimmt bei dieser Drehbewegung die Adapterstruktur 36 mit, bis das Eingriffselement 12 des Entkopplungsglieds 11' an dem Ende der Führungskurve 36a auf Anschlag gelangt. Die Drehbewegung des Gehäuseteils 1 ist vorzugsweise erst in dessen axialen Anschlagposition möglich, wofür eine bis in die Anschlagposition wirksame Verdrehsicherung auch zwischen den Gehäuseteilen 1 und 4 gebildet sein kann.

Die im Führungseingriff bewirkte Bewegung des Entkopplungsglieds 11' weist eine axiale Länge auf, die größer ist als die Länge X der vollständigen Kupplungsbewegung. Das Entkopplungsglied 11' drückt bei seiner. Entkopplungsbewegung das Kupplungseingangsglied 6' über dessen im Ruhezustand eingenommene Halteposition hinaus und blockiert es in dieser Entkopplungsposition. Das Kupplungseingangsglied 6' nimmt bei dieser erzwungenen Entkopplungsbewegung über das Anschlagelement 29b das Auslöseelement 28 mit. Über die Verhakung der Mitnehmer 29c und 34a wird das Sperrglied 34 gegen die Kraft der Sperrfeder 31 ebenfalls mitgenommen und aus dem Sperreingriff bewegt. Das Sperrglied 24 kann dem Sperrglied 34 nicht folgen, da es gegen das Gehäuseteil 4 auf Anschlag ist. Durch das Lösen der Gehäuseteile 1 und 4 wird somit mittels des Entkopplungsmechanismus, den die Gehäuseteile 1 und 4 über die Adapterstruktur 35 mit dem Entkopplungsglied 11' bilden, die Verdrehsperre gelöst. Sollte das Kupplungseingangsglied 6' noch nicht die Nulldosisposition einnehmen, wird es von dem Antriebsglied 25 spätestens jetzt in die Nulldosisposition verdreht und die Dosisanzeige 20" genullt. In diesem Zusammenhang sei wiederholt auf den besonderen Vorteil der Kopplung zwischen der Dosisanzeige 20" und dem Kupplungseingangsglied 6' hingewiesen, dass nämlich bei jeder Ausschüttung die Dosisanzeige 20" der ausgeschütteten Dosis entsprechend zurückgesetzt wird. Sollte die eingestellte Dosis einmal nicht ausgeschüttet worden sein, weil beispielsweise der Injektionsvorgang abgebrochen wurde oder das Behältnis 2 nicht die eingestellte Dosis vollständig mehr enthielt, kann der Benutzer dies an der in diesem Fall nur teilweise zurückgesetzten Dosisanzeige 20" ablesen.

### Bezugszeichen:

- 1: erstes Gehäuseteil
- 1a: Führungskurve
- 2: Behältnis
- 3: Kolben
- 4: zweites Gehäuseteil
- 4a: Linearführung
- 4b: Linearführung
- 4c: Umfangsführung
- 4d: Fixierelement
- 4e: Axialführung
- 5: Antriebsglied
- 6,6': Kupplungseingangsglied
- 6a: Eingriffselement
- 6b: Eingriffselement
- 6c: Stützstruktur
- 6d: Stützstruktur
- 7,7': Kupplungszwischenglied
- 7a: Eingriffselement
- 7b: Eingriffselement
- 7c: Eingriffselement
- 8, 8': Kupplungshülse
- 8a: Eingriffselement
- 9: Kupplungsausgangsglied
- 10: Kupplungs-Rückstellglied
- 11, 11': Entkopplungsglied
- 11a: Eingriffselement
- 12: Eingriffselement
- 13: Fixierelement
- 14: Kupplungs-Rückstellglied
- 15: Kolbenstange
- 15a: Teller
- 16: Auslöseelement
- 17: Ausgleichsfeder
- 18, 18': Dosierglied
- 18a: Eingriffselement
- 19: Kupplungs-Rückstellglied
- 20, 20' 20": Dosisanzeige
- 21: Anzeigekopplungsglied
- 22: Anzeigekopplungsglied
- 23: Anzeigekopplungsglied
- 24: erstes Sperrglied
- 24a: Sperrzähne
- 24b: Gewinde
- 24c: Verdrehanschlag
- 24d: Verdrehanschlag
- 25: Antriebsglied
- 26: Stützstruktur
- 27: Stoppglied
- 28: Auslöseelement
- 29: Innenteil
- 29a: Verzahnung
- 29b: Anschlagelement
- 29c: Mitnehmer
- 30: Gehäuseteil
- 31: Sperrfeder
- 32: Feder
- 33: Verbindungsteil
- 33a: Verzahnung
- 34: zweites Sperrglied
- 34a: Mitnehmer
- 35: Adapterstruktur
- 35a: Führungskurve
- 36: -
- 37: Schutzkappe
- 38: Gehäuseteil
- 39: Nadelschutz

## Patentansprüche

1. Vorrichtung für die dosierte Verabreichung eines fluiden Produkts, die Vorrichtung umfassend:
a) ein Gehäuse (4) mit einem Reservoir für das Produkt,
b) eine Fördereinrichtung (3, 15) für das Produkt,
c) ein durch Einstellung einer Produktdosis beeinflusstes Antriebsglied (5; 25),
d) ein mit dem Antriebsglied (5; 25) gekoppeltes Kupplungseingangsglied (6; 6'),
e) ein mit der Fördereinrichtung (3, 15) gekoppeltes Kupplungsausgangsglied (9) f) und eine Halteeinrichtung (7, 9, 10; 6c, 14, 18'; 17, 19, 23, 24), die die Kupplungsglieder (6, 9; 6', 9) in einer voneinander entkoppelten Halteposition hält,
g) wobei wenigstens eines der Kupplungsglieder (6, 9; 6', 9) mit einer Kupplungsbewegung (X) aus der Halteposition in einen Kupplungseingriff bewegbar ist, in dem eine Antriebskraft des Antriebsglieds (5; 25) über die Kupplungsglieder (6, 9; 6', 9) eine Ausschüttbewegung der Fördereinrichtung (3, 15) bewirkt,
**dadurch gekennzeichnet, dass**
h) das Kupplungsausgangsglied (9) in der Halteposition der Kupplungsglieder (6, 9; 6', 9) relativ zu dem Gehäuse (1,4) so fixiert ist, dass es keine die Ausschüttbewegung bewirkende Bewegung ausführen kann.

2. Vorrichtung nach Anspruch 1, **dadurch gekennzeichnet, dass** die Halteeinrichtung ein Rückstellglied (10; 14; 17, 19) umfasst und das Rückstellglied (10; 14; 17, 19) das wenigstens eine, die Kupplungsbewegung (X) ausführende Kupplungsglied (6; 6') mit einer der Kupplungsbewegung (X) entgegenwirkenden, elastischen Rückstellkraft beaufschlagt.

3. Vorrichtung nach dem vorhergehenden Anspruch, **dadurch gekennzeichnet, dass** das Rückstellglied (14; 19) gegen die Richtung der Kupplungsbewegung (X) an dem Kupplungseingangsglied (6') oder einer Struktur (6c; 23) abgestützt ist, die zumindest in der Halteposition der Kupplungsglieder (6, 9; 6', 9) gegen die Richtung der Kupplungsbewegung (X) nicht beweglich oder nur bis auf einen Anschlag 33 beweglich mit dem Kupplungseingangsglied (6') verbunden ist.

4. Vorrichtung nach einem der zwei vorhergehenden Ansprüche, **dadurch gekennzeichnet, dass** das Rückstellglied (14; 19) in Richtung der Kupplungsbewegung (X) an dem Gehäuse (1, 4) oder einer zumindest in der Halteposition der Kupplungsglieder (6, 9; 6', 9) in Richtung der Kupplungsbewegung (X) nicht beweglich mit dem zweiten Gehäuseteil (4) verbundenen Struktur (18'; 23) abgestützt ist.

5. Vorrichtung nach einem der Ansprüche 2 und 3, **dadurch gekennzeichnet, dass** das Rückstellglied (17) in Richtung der Kupplungsbewegung (X) an einem Förderglied (15) der Fördereinrichtung (3, 15) abgestützt ist.

6. Vorrichtung nach einem der Ansprüche 2 und 3, **dadurch gekennzeichnet, dass** das Rückstellglied (10) in Richtung der Kupplungsbewegung (X) an dem Kupplungsausgangsglied (9) oder einem Verbindungsteil abgestützt ist, wobei das Verbindungsteil zumindest in der Halteposition der Kupplungsglieder (6, 9; 6', 9) in die Richtung der Kupplungsbewegung (X) nicht beweglich mit dem Kupplungsausgangsglied (9) verbunden ist.

7. Vorrichtung nach einem der vorhergehenden Ansprüche, ferner umfassend ein Kupplungszwischenglied (7; T), das in einem Sperreingriff das Kupplungsausgangsglied (9) relativ zu dem Gehäuse (1, 4) fixiert.

8. Vorrichtung nach dem vorhergehenden Anspruch, **dadurch gekennzeichnet, dass** das Kupplungszwischenglied (7; 7') in Richtung der Kupplungsbewegung (X) aus dem Sperreingriff bewegbar ist.

9. Vorrichtung nach einem der zwei vorhergehenden Ansprüche in Kombination mit Anspruch 2, **dadurch gekennzeichnet, dass** das Rückstellglied (10) über das Kupplungszwischenglied (7) auf das Kupplungseingangsglied (6) wirkt.

10. Vorrichtung nach einem der vorhergehenden Ansprüche, ferner umfassend ein mit dem Kupplungseingangsglied (6; 6') gekoppeltes, in der Halteposition der Kupplungsglieder (6, 9; 6', 9) von der Fördereinrichtung (3, 15) entkoppeltes Dosierglied (18; 18'; 28) für eine Einstellung der Produktdosis.

11. Vorrichtung nach einem der vorhergehenden Ansprüche, ferner umfassend ein Dosierglied (18; 18'; 28) für eine Einstellung einer Produktdosis, das über das Kupplungseingangsglied (6; 6') so mit dem Antriebsglied (5; 25) gekoppelt ist, dass durch eine für die Einstellung der Dosis von dem Dosierglied (18; 18'; 28) ausgeführte Dosierbewegung das Antriebsglied (5; 25) mit einer der Antriebskraft entgegen gerichteten Kraft beaufschlagt wird.

12. Vorrichtung nach einem der vorhergehenden Ansprüche, ferner umfassend eine mit dem Kupplungseingangsglied (6; 6') gekoppelte, in der Halteposition der Kupplungsglieder (6, 9; 6', 9) von der Fördereinrichtung (3, 15) entkoppelte Dosisanzeige (20; 20'; 20") zum Anzeigen einer einstellbaren Produktdosis.

13. Vorrichtung nach einem der vorhergehenden Ansprüche, **dadurch gekennzeichnet, dass** das Antriebsglied (5) relativ zu dem Gehäuse (1, 4) translatorisch bewegbar und mit dem Kupplungseingangsglied (6; 6') und dem Gehäuse (1, 4) so gekoppelt ist, dass eine translatorische Antriebsbewegung des Antriebsglieds (5) eine Rotationsbewegung des Kupplungseingangsglieds (6; 6') bewirkt.

14. Vorrichtung nach dem vorhergehenden Anspruch, **dadurch gekennzeichnet, dass** das Antriebsglied (5) mit dem Kupplungseingangsglied (6; 6') oder dem Gehäuse (1, 4) in einem Gewindeeingriff ist.

15. Vorrichtung nach einem der zwei vorhergehenden Ansprüche, **dadurch gekennzeichnet, dass** das Kupplungseingangsglied (6; 6') die Kupplungsbewegung (X) ausführt und das Antriebsglied (5) mit dem Kupplungseingangsglied (6; 6') so gekoppelt ist, dass die Antriebsbewegung des Antriebsglieds (5) die Kupplungsbewegung (X) bewirkt.

16. Vorrichtung nach dem vorhergehenden Anspruch, **dadurch gekennzeichnet, dass** das Antriebsglied (5) bei der Antriebsbewegung das Kupplungseingangsglied (6; 6') bis in den Kupplungseingriff mitnimmt.

17. Vorrichtung nach einem der vorhergehenden Ansprüche, **dadurch gekennzeichnet, dass** das Antriebsglied (5) verdrehgesichert linear geführt wird.

18. Vorrichtung nach einem der vorhergehenden Ansprüche, **dadurch gekennzeichnet, dass** das Antriebsglied (5; 25) das Kupplungseingangsglied (6; 6') rotatorisch antreibt.

19. Vorrichtung nach einem der vorhergehenden Ansprüche, **dadurch gekennzeichnet, dass** das Kupplungseingangsglied (6; 6') für die Ausführung der Kupplungsbewegung (X) axial bewegbar ist.

20. Vorrichtung nach einem der Ansprüche 1 bis 13, **dadurch gekennzeichnet, dass** das Antriebsglied (25) ein durch Einstellung einer Produktdosis spannbares Federglied ist.

21. Vorrichtung nach dem vorhergehenden Anspruch, **dadurch gekennzeichnet, dass** das Antriebsglied (25) eine Spiralfeder mit einer inneren Federwindung und wenigstens einer die innere Federwindung umgebenden äusseren Federwindung ist.

22. Vorrichtung nach einem der vorhergehenden Ansprüche, ferner umfassend ein Auslöseelement (28), das bei einer Betätigung eine Auslösebewegung ausführt und mit dem wenigstens einen der Kupplungsglieder (6, 9; 6', 9), das die Kupplungsbewegung (X) ausführt, so gekoppelt ist, dass die Auslösebewegung des Auslöseelements (28) die Kupplungsbewegung (X) bewirkt.

23. Vorrichtung nach dem vorhergehenden Anspruch, **dadurch gekennzeichnet, dass** das Auslöseelement (28) bei der Auslösebewegung das wenigstens eine die Kupplungsbewegung (X) ausführende Kupplungsglied (6') bis in den Kupplungseingriff mitnimmt.

24. Vorrichtung nach einem der zwei vorhergehenden Ansprüche, **dadurch gekennzeichnet, dass** das Auslöseelement (28) in einem proximalen Endbereich des Gehäuses (1, 4) angeordnet ist, vorzugsweise koaxial zu dem Gehäuse (1, 4), und die Auslösebewegung relativ zu dem Gehäuse (1, 4) in die distale Richtung erfolgt.

25. Vorrichtung nach einem der drei vorhergehenden Ansprüche, **dadurch gekennzeichnet, dass** das Auslöseelement (28) ein Dosierglied für die Einstellung der Produktdosis bildet.

26. Vorrichtung nach dem vorhergehenden Anspruch, **dadurch gekennzeichnet, dass** das Auslöseelement (28) bei der Einstellung der Produktdosis eine Dosierbewegung ausführt und mit dem Kupplungseingangs glied (6') so gekoppelt ist, dass die Dosierbewegung des Auslöseelements (28) eine Dosierdrehbewegung des Kupplungseingangsglieds (6') bewirkt.

27. Vorrichtung nach einem der fünf vorhergehenden Ansprüche, **dadurch gekennzeichnet, dass** das Auslöseelement (28) das Gehäuse (1, 4) in dem Endbereich umgibt und relativ zu dem Gehäuse (1, 4) rotatorisch bewegbar ist.

## Claims

1. A device for the dosed administration of a fluid product, the device including:
a) a housing (4) having a reservoir for the product,
b) a conveying device (3, 15) for the product,
c) a drive member (5; 25) influenced by setting of a product dose,
d) a coupling input member (6; 6') coupled to the drive member (5; 25),
e) a coupling output member (9) coupled to the conveying device (3, 15), and
f) a holding device (7, 9, 10; 6c, 14, 18'; 17, 19, 23, 24) which holds the coupling members (6, 9; 6', 9) in a mutually uncoupled holding position,
g) wherein at least one of the coupling members (6, 9; 6', 9) is moveable with a coupling movement (X) out of the holding position into a coupling engagement in which a drive force of the drive member (5; 25) causes a dispensing movement of the conveying device (3, 15) by way of the coupling members (6, 9; 6', 9),
**characterised in that**
h) the coupling output member (9) is fixed in the holding position of the coupling members (6, 9; 6', 9) relative to the housing (1, 4) such that it cannot perform any movement causing the dispensing movement.

2. A device according to claim 1 **characterised in that** the holding device includes a return member (10; 14; 17, 19) and the return member (10; 14; 17, 19) acts on the at least one coupling member (6; 6') performing the coupling movement (X) with a resilient return force counteracting the coupling movement (X).

3. A device according to the preceding claim **characterised in that** the return member (14; 19) is supported in opposite relationship to the direction of the coupling movement (X) on the coupling input member (6') or a structure (6c; 23) which is connected to the coupling input member (6') non-moveably in opposite relationship to the direction of the coupling movement (X) or moveable only as far as an abutment (33) at least in the holding position of the coupling members (6, 9; 6', 9).

4. A device according to one of the two preceding claims **characterised in that** the return member (14; 19) is supported in the direction of the coupling movement (X) at the housing (1, 4) or a structure (18'; 23) which is non-moveably connected to the second housing portion (4) in the direction of the coupling movement (X) at least in the holding position of the coupling members (6, 9; 6', 9).

5. A device according to one of claims 2 and 3 **characterised in that** the return member (17) is supported in the direction of the coupling movement (X) at a conveying member (15) of the conveying device (3, 15).

6. A device according to one of claims 2 and 3 **characterised in that** the return member (10) is supported in the direction of the coupling movement (X) at the coupling output member (9) or a connecting portion, wherein the connecting portion is non-moveably connected to the coupling output member (9) at least in the holding position of the coupling members (6, 9; 6', 9) in the direction of the coupling movement (X).

7. A device according to one of the preceding claims further including a coupling intermediate member (7; T) which in a locking engagement fixes the coupling output member (9) relative to the housing (1, 4).

8. A device according to the preceding claim **characterised in that** the coupling intermediate member (7; 7') is moveable out of the locking engagement in the direction of the coupling movement (X).

9. A device according to one of the two preceding claims in combination with claim 2 **characterised in that** the return member (10) acts on the coupling input member (6) by way of the coupling intermediate member (7).

10. A device according to one of the preceding claims further including a dosing member (18; 18'; 28) for setting of the product dose, that is coupled to the coupling input member (6; 6') and is uncoupled from the conveying device (3, 15) in the holding position of the coupling members (6, 9; 6', 9).

11. A device according to one of the preceding claims and further including a dosing member (18; 18'; 28) for setting of a product dose, that is coupled by way of the coupling input member (6; 6') to the drive member (5; 25) such that the drive member (5; 25) is acted upon with a force directed in opposite relationship to the drive force by a dosing movement carried out for setting the dose by the dosing member (18; 18'; 28).

12. A device according to one of the preceding claims and further including a dose display (20; 20'; 20") for displaying a settable product dose, that is coupled to the coupling input member (6; 6') and uncoupled from the conveying device (3, 15) in the holding position of the coupling members (6, 9; 6', 9).

13. A device according to one of the preceding claims **characterised in that** the drive member (5) is moveable with a translatory movement relative to the housing (1, 4) and is so coupled to the coupling input member (6; 6') and the housing (1, 4) that a translatory drive movement of the drive member (5) causes a rotational movement of the coupling input member (6; 6').

14. A device according to the preceding claim **characterised in that** the drive member (5) is in threaded engagement with the coupling input member (6; 6') or the housing (1, 4).

15. A device according to one of the two preceding claims **characterised in that** the coupling input member (6; 6') performs the coupling movement and the drive member (5) is so coupled to the coupling input member (6; 6') that the drive movement of the drive member (5) causes the coupling movement (X).

16. A device according to the preceding claim **characterised in that** the drive member (5) in the drive movement entrains the coupling input member (6; 6') into the coupling engagement.

17. A device according to one of the preceding claims **characterised in that** the drive member (5) is linearly guided in non-rotatable relationship.

18. A device according to one of the preceding claims **characterised in that** the drive member (5; 25) rotationally drives the coupling input member (6; 6').

19. A device according to one of the preceding claims **characterised in that** the coupling input member (6; 6') is axially moveable for performing the coupling movement (X).

20. A device according to one of claims 1 to 13 **characterised in that** the drive member (25) is a spring member which can be stressed by setting a product dose.

21. A device according to the preceding claim **characterised in that** the drive member (25) is a coil spring having an inner spring turn and at least one outer spring turn surrounding the inner spring turn.

22. A device according to one of the preceding claims and further including a triggering element (28) which upon actuation performs a triggering movement and is coupled to the at least one of the coupling members (6, 9; 6', 9) which performs the coupling movement (X) in such a way that the triggering movement of the triggering element (28) causes the coupling movement (X).

23. A device according to the preceding claim **characterised in that** in the triggering movement the triggering element (28) entrains the at least one coupling member (6') performing the coupling movement (X) into the coupling engagement.

24. A device according to one of the two preceding claims **characterised in that** the triggering element (28) is arranged in a proximal end region of the housing (1, 4), preferably coaxially with the housing (1, 4), and the triggering movement relative to the housing (1, 4) is effected in the distal direction.

25. A device according to one of the three preceding claims **characterised in that** the triggering element (28) forms a dosing member for setting the product dose.

26. A device according to the preceding claim **characterised in that** when setting the product dose the triggering element (28) performs a dosing movement and is so coupled to the coupling input member (6') that the dosing movement of the triggering element (28) causes a rotary dosing movement of the coupling input member (6').

27. A device according to one of the five preceding claims **characterised in that** the triggering element (28) surrounds the housing (1, 4) in the end region and is rotationally moveable relative to the housing (1, 4).

## Revendications

1. Dispositif d'administration dosée d'un produit fluide, le dispositif comprenant :
a) un boîtier (4) avec un réservoir pour le produit,
b) un système de refoulement (3, 15) pour le produit,
c) un organe d'entraînement (5 ; 25) influencé par le réglage d'une dose de produit,
d) un organe d'entrée de couplage (6 ; 6') couplé à l'organe d'entraînement (5 ; 25),
e) un organe de sortie de couplage (9) couplé au système de refoulement (3, 15), et
f) un système de maintien (7, 9, 10 ; 6c, 14, 18' ; 17, 19, 23, 24), qui maintient les organes de couplage (6, 9 ; 6', 9) dans une position de maintien découplée les uns des autres,
g) dans lequel au moins un des organes de couplage (6, 9 ; 6', 9) peut être déplacé avec un déplacement de couplage (X) depuis la position de maintien dans une prise par couplage, dans laquelle une force d'entraînement de l'organe d'entraînement (5 ; 25) entraîne par l'intermédiaire des organes de couplage (6, 9 ; 6', 9) un déplacement de déversement du système de refoulement (3, 15),
**caractérisé en ce que**
h) l'organe de sortie de couplage (9) est fixé dans la position de maintien des organes de couplage (6, 9 ; 6', 9) par rapport au boîtier (1, 4) de telle sorte qu'il ne peut exécuter aucun déplacement entraînant le déplacement de déversement.

2. Dispositif selon la revendication 1, **caractérisé en ce que** le système de maintien comprend un organe de rappel (10 ; 14 ; 17, 19) et l'organe de rappel (10 ; 14 ; 17, 19) soumet l'au moins un organe de couplage (6 ; 6') exécutant le déplacement de couplage (X) à l'action d'une force de rappel élastique contrecarrant le déplacement de couplage (X).

3. Dispositif selon la revendication précédente, **caractérisé en ce que** l'organe de rappel (14 ; 19) prend appui dans le sens opposé à la direction du déplacement de couplage (X) au niveau de l'organe d'entrée de couplage (6') ou au niveau d'une structure (6c ; 23), qui est reliée à l'organe d'entrée de couplage (6') de manière non mobile au moins dans la position de maintien des organes de couplage (6, 9 ; 6', 9) dans le sens opposé à la direction du déplacement de couplage (X) ou de manière mobile seulement jusqu'à une butée (33).

4. Dispositif selon l'une des deux revendications précédentes, **caractérisé en ce que** l'organe de rappel (14 ; 19) prend appui dans la direction du déplacement de couplage (X) au niveau du boîtier (1, 4) ou d'une structure (18' ; 23) reliée à la deuxième partie de boîtier (4) de manière non mobile en direction du déplacement de couplage (X) au moins dans la position de maintien des organes de couplage (6, 9 ; 6', 9).

5. Dispositif selon l'une quelconque des revendications 2 et 3, **caractérisé en ce que** l'organe de rappel (17) prend appui dans la direction du déplacement de couplage (X) au niveau d'un organe de refoulement (15) du système de refoulement (3, 15).

6. Dispositif selon l'une quelconque des revendications 2 et 3, **caractérisé en ce que** l'organe de rappel (10) prend appui dans la direction du déplacement de couplage (X) au niveau de l'organe de sortie de couplage (9) ou d'une partie de liaison, dans lequel la partie de liaison est reliée à l'organe de sortie de couplage (9) de manière non mobile dans la direction du déplacement de couplage (X) au moins dans la position de maintien des organes de couplage (6, 9 ; 6', 9).

7. Dispositif selon l'une quelconque des revendications précédentes, comprenant en outre un organe intermédiaire de couplage (7 ; T), qui fixe dans une prise par verrouillage l'organe de sortie de couplage (9) par rapport au boîtier (1, 4).

8. Dispositif selon la revendication précédente, **caractérisé en ce que** l'organe intermédiaire de couplage (7 ; 7') peut être déplacé hors de la prise par verrouillage en direction du déplacement de couplage (X).

9. Dispositif selon l'une quelconque des deux revendications précédentes en combinaison avec la revendication 2, **caractérisé en ce que** l'organe de rappel (10) agit sur l'organe d'entrée de couplage (6) par l'intermédiaire de l'organe intermédiaire de couplage (7).

10. Dispositif selon l'une quelconque des revendications précédentes, comprenant en outre un organe de dosage (18 ; 18' ; 28) couplé à l'organe d'entrée de couplage (6 ; 6'), découplé dans la position de maintien des organes de couplage (6, 9 ; 6', 9) du système de refoulement (3, 15) pour un réglage de la dose de produit.

11. Dispositif selon l'une quelconque des revendications précédentes, comprenant en outre un organe de dosage (18 ; 18' ; 28) pour un réglage d'une dose de produit, qui est couplé par l'intermédiaire de l'organe d'entrée de couplage (6 ; 6') de telle sorte à l'organe d'entraînement (5 ; 25) que l'organe d'entraînement (5 ; 25) est soumis à l'action d'une force dirigée dans le sens opposé à la force d'entraînement par un déplacement de dosage exécuté pour le réglage de la dose par l'organe de dosage (18 ; 18' ; 28).

12. Dispositif selon l'une quelconque des revendications précédentes, comprenant en outre un affichage de dose (20 ; 20' ; 20") couplé à l'organe d'entrée de couplage (6 ; 6'), découplé dans la position de maintien des organes de couplage (6, 9 ; 6', 9) du système de refoulement (3, 15), servant à afficher une dose de produit pouvant être réglée.

13. Dispositif selon l'une quelconque des revendications précédentes, **caractérisé en ce que** l'organe d'entraînement (5) peut être déplacé par translation par rapport au boîtier (1, 4) et est couplé à l'organe d'entrée de couplage (6 ; 6') et au boîtier (1, 4) de telle sorte qu'un déplacement d'entraînement par translation de l'organe d'entraînement (5) entraîne un déplacement en rotation de l'organe d'entrée de couplage (6 ; 6').

14. Dispositif selon la revendication précédente, **caractérisé en ce que** l'organe d'entraînement (5) est en prise par filetage avec l'organe d'entrée de couplage (6 ; 6') ou le boîtier (1, 4).

15. Dispositif selon l'une quelconque des deux revendications précédentes, **caractérisé en ce que** l'organe d'entrée de couplage (6 ; 6') exécute le déplacement de couplage (X) et l'organe d'entraînement (5) est couplé à l'organe d'entrée de couplage (6 ; 6') de telle sorte que le déplacement d'entraînement de l'organe d'entraînement (5) entraîne le déplacement de couplage (X).

16. Dispositif selon la revendication précédente, **caractérisé en ce que** l'organe d'entraînement (5) entraîne lors du déplacement d'entraînement l'organe d'entrée de couplage (6 ; 6') jusque dans la prise par couplage.

17. Dispositif selon l'une quelconque des revendications précédentes, **caractérisé en ce que** l'organe d'entraînement (5) est guidé linéairement de manière bloquée empêchant toute rotation.

18. Dispositif selon l'une quelconque des revendications précédentes, **caractérisé en ce que** l'organe d'entraînement (5 ; 25) entraîne de manière rotative l'organe d'entrée de couplage (6 ; 6').

19. Dispositif selon l'une quelconque des revendications précédentes, **caractérisé en ce que** l'organe d'entrée de couplage (6 ; 6') peut être déplacé de manière axiale pour l'exécution du déplacement de couplage (X).

20. Dispositif selon l'une quelconque des revendications 1 à 13, **caractérisé en ce que** l'organe d'entraînement (25) est un organe de ressort pouvant être tendu par le réglage d'une dose de produit.

21. Dispositif selon la revendication précédente, **caractérisé en ce que** l'organe d'entraînement (25) est un ressort hélicoïdal avec une spire de ressort intérieure et au moins une spire de ressort extérieure entourant la spire de ressort intérieure.

22. Dispositif selon l'une quelconque des revendications précédentes, comprenant en outre un élément de déclenchement (28), qui exécute lors d'un actionnement un déplacement de déclenchement et est couplé à l'au moins un des organes de couplage (6, 9 ; 6', 9), qui exécute le déplacement de couplage (X), de telle sorte que le déplacement de déclenchement de l'élément de déclenchement (28) entraîne le déplacement de couplage (X).

23. Dispositif selon la revendication précédente, **caractérisé en ce que** l'élément de déclenchement (28) entraîne lors du déplacement de déclenchement l'au moins un organe de couplage (6') exécutant le déplacement de couplage (X) jusque dans la prise par couplage.

24. Dispositif selon l'une quelconque des deux revendications précédentes, **caractérisé en ce que** l'élément de déclenchement (28) est disposé dans une zone d'extrémité proximale du boîtier (1, 4), de préférence de manière coaxiale par rapport au boîtier (1, 4), et le déplacement de déclenchement est effectué dans la direction distale par rapport au boîtier (1, 4).

25. Dispositif selon l'une quelconque des trois revendications précédentes, **caractérisé en ce que** l'élément de déclenchement (28) forme un organe de dosage pour le réglage de la dose de produit.

26. Dispositif selon la revendication précédente, **caractérisé en ce que** l'élément de déclenchement (28) exécute lors du réglage de la dose de produit un déplacement de dosage et est couplé à l'organe d'entrée de couplage (6') de telle sorte que le déplacement de dosage de l'élément de déclenchement (28) entraîne un déplacement de rotation de dosage de l'organe d'entrée de couplage (6').

27. Dispositif selon l'une quelconque des cinq revendications précédentes, **caractérisé en ce que** l'élément de déclenchement (28) entoure le boîtier (1, 4) dans la zone d'extrémité et peut être déplacé en rotation par rapport au boîtier (1, 4).
